# EUROPEAN PATENT SPECIFICATION

(11) **EP 1 716 145 B1**
(45) Date of publication and mention of the grant of the patent: **27.08.2008**
(21) Application number: 05708289.3
(22) Date of filing: 11.02.2005
(51) Int. Cl.: C07D 455/06, A61K 31/473, A61P 25/14

(54) **DIHYDROTETRABENAZINES AND PHARMACEUTICAL COMPOSITIONS CONTAINING THEM**
DIHYDROTETRABENAZINE UND DIESE ENTHALTENDE PHARMAZEUTISCHE ZUSAMMENSETZUNGEN
DIHYDROTETRABENAZINES ET COMPOSITIONS PHARMACEUTIQUES LES CONTENANT

(30) Priority: 11.02.2004 GB 0403037; 11.02.2004 US 543531 P
(43) Date of publication of application: 02.11.2006
(73) Proprietor: Cambridge Laboratories (Ireland) Limited, Dublin 4 (IE)
(72) Inventor: CLARKE, Ian, Northampton, Northamptonshire NN1 5JE (GB); TURTLE, Robert, Pagnell, Buckinghamshire MK16 0JG (GB); JOHNSTON, Grant, Northampton, Northamptonshire NN1 5LN (GB)
(74) Representative: Hutchins, Michael Richard
(86) International application number: PCT/GB2005/000464
(87) International publication number: WO 2005/077946

(56) References cited:
- US-A- 2 830 993
- US-A- 2 843 591
- US-A- 6 087 376
- M. R. KILBOURN ET AL: "Absolute Configuration of (+)-alpha-Dihydrotetrabenazine, an Active Metabolite of Tetrabenazine" CHIRALITY, vol. 9, no. 1, 1997, pages 59-62, XP002329921 cited in the application
- M. KILBOURN ET AL: "Binding of alpha-dihydrotetrabenazine to the vesicular monoamine transporter is stereospecific" EUROPEAN JOURNAL OF PHARMACOLOGY, vol. 278, no. 3, 1995, pages 249-252, XP002329922 cited in the application
- A. BROSSI ET AL: "Syntheseversuche in der Emetin-Reihe. 3. 2-Hydroxy-hydrobenzo[a]chinolizine" HELVETICA CHIMICA ACTA, vol. 41, 1958, pages 1793-1806, XP008047475 BASEL, CH cited in the application

## Description

This invention relates to novel dihydrotetrabenazine isomers, pharmaceutical compositions containing them, processes for making them and their therapeutic uses.

### Background of the Invention

Tetrabenazine (Chemical name: 1,3,4,6,7,11b-hexahydro-9,10-dimethoxy-3-(2-methylpropyl)-2H-benzo(a)quinolizin-2-one) has been in use as a pharmaceutical drug since the late 1950s. Initially used as an anti-psychotic, tetrabenazine is currently used for treating hyperkinetic movement disorders such as Huntington's disease, hemiballismus, senile chorea, tic, tardive dyskinesia and Tourette's syndrome, see for example Jankovic et al., Am. J. Psychiatry. (1999) Aug; 156(8):1279-81 and Jankovic et al., Neurology (1997) Feb; 48(2):358-62.

The primary pharmacological action of tetrabenazine is to reduce the supply of monoamines (e.g. dopamine, serotonin, and norepinephrine) in the central nervous system by inhibiting the human vesicular monoamine transporter isoform 2 (hVMAT2). The drug also blocks postsynaptic dopamine receptors.

Tetrabenazine is an effective and safe drug for the treatment of a variety of hyperkinetic movement disorders and, in contrast to typical neuroleptics, has not been demonstrated to cause tardive dyskinesia. Nevertheless, tetrabenazine does exhibit a number of dose-related side effects including causing depression, parkinsonism, drowsiness, nervousness or anxiety, insomnia and, in rare cases, neuroleptic malignant syndrome.

The central effects of tetrabenazine closely resemble those of reserpine, but it differs from reserpine in that it lacks activity at the VMAT1 transporter. The lack of activity at the VMAT1 transporter means that tetrabenazine has less peripheral activity than reserpine and consequently does not produce VMAT1-related side effects such as hypotension.

The chemical structure of tetrabenazine is as shown in Figure 1 below.

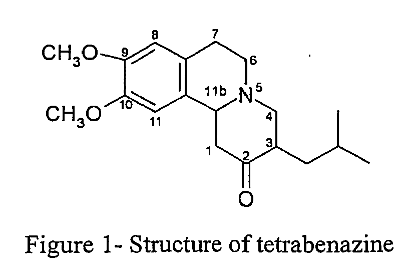

The compound has chiral centres at the 3 and 11b carbon atoms and hence can, theoretically, exist in a total of four isomeric forms, as shown in Figure 2. 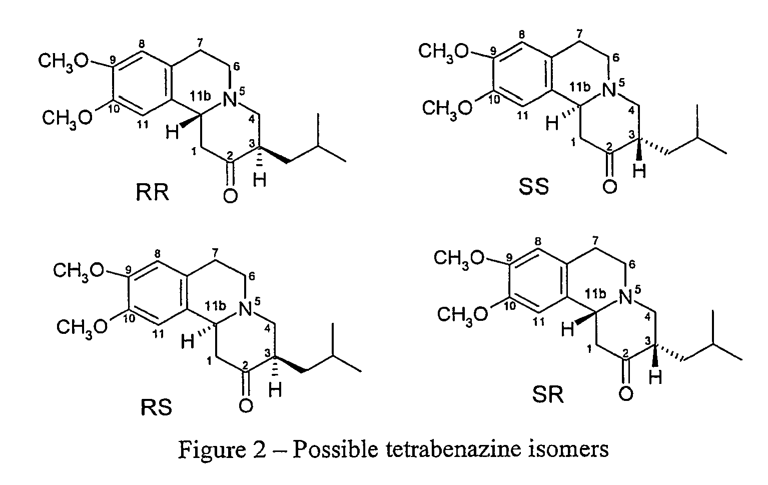 In Figure 2, the stereochemistry of each isomer is defined using the "R and S" nomenclature developed by Cahn, Ingold and Prelog, see Advanced Organic Chemistry by Jerry March, 4th Edition, John Wiley & Sons, New York, 1992, pages 109-114. In Figure 2 and elsewhere in this patent application, the designations "R" or "S" are given in the order of the position numbers of the carbon atoms. Thus, for example, *RS* is a shorthand notation for 3*R*,11b*S*. Similarly, when three chiral centres are present, as in the dihydrotetrabenazines described below, the designations "R" or "*S*" are listed in the order of the carbon atoms 2, 3 and 11b. Thus, the 2*S*,3*R*,*11bR* isomer is referred to in short hand form as *SRR* and so on.

Commercially available tetrabenazine is a racemic mixture of the *RR* and *SS* isomers and it would appear that the *RR* and *SS* isomers (hereinafter referred to individually or collectively as *trans*-tetrabenazine because the hydrogen atoms at the 3 and 11b positions have a *trans* relative orientation) are the most thermodynamically stable isomers.

Tetrabenazine has somewhat poor and variable bioavailability. It is extensively metabolised by first-pass metabolism, and little or no unchanged tetrabenazine is typically detected in the urine. The major metabolite is dihydrotetrabenazine (Chemical name: 2-hydroxy-3-(2-methylpropyl)-1,3,4,6,7,11b-hexahydro-9,10-dimethoxy-benzo(a)quinolizine) which is formed by reduction of the 2-keto group in tetrabenazine, and is believed to be primarily responsible for the activity of the drug (see Mehvar et al., Drug Metab.Disp, 15, 250-255 (1987) and J. Pharm. Sci., 76, No.6, 461-465 (1987)).

Four dihydrotetrabenazine isomers have previously been identified and characterised, all of them being derived from the more stable *RR* and *SS* isomers of the parent tetrabenazine and having a *trans* relative orientation between the hydrogen atoms at the 3 and 11b positions) (see Kilbourn et al., Chirality, 9:59-62 (1997) and Brossi et al., Helv. Chim. Acta., vol. XLI, No. 193, pp1793-1806 (1958). The four isomers are (+)-α-dihydrotetrabenazine, (-)-α-dihydrotetrabenazine, (+)-β-dihydrotetrabenazine and (-)-β-dihydrotetrabenazine. The structures of the four known dihydrotetrabenazine isomers are considered to be as shown in Figure 3. 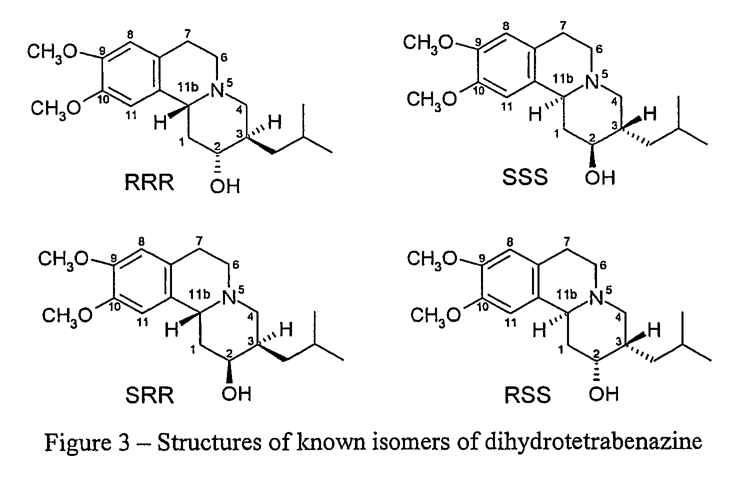 Kilbourn et al.,(see Eur. J. Pharmacol., 278:249-252 (1995 ) and Med. Chem. Res., 5:113-126 (1994)) investigated the specific binding of individual radio-labelled dihydrotetrabenazine isomers in the conscious rat brain. They found that the (+)-α-[¹¹C]dihydrotetrabenazine (2*R*,3*R*,11b*R*) isomer accumulated in regions of the brain associated with higher concentrations of the neuronal membrane dopamine transporter (DAT) and the vesicular monoamine transporter (VMAT2). However, the essentially inactive (-)-α-[¹¹C]dihydrotetrabenazine isomer was almost uniformly distributed in the brain, suggesting that specific binding to DAT and VMAT2 was not occurring. The *in vivo* studies correlated with *in vitro* studies which demonstrated that the (+)-α-[¹¹C]dihydrotetrabenazine isomer exhibits a Kᵢ for [³H]methoxytetrabenazine >2000-fold higher than the Kᵢ for the (-)-α-[¹¹C]dihydrotetrabenazine isomer.

US 6,087,376 (Crooks et al) discloses the use of lobeline for the treatment of CNS diseases. Lobeline is described as having the ability to inhibit [³H]dihydrotetrabenazine binding to VMAT2.

To date, so far as the applicants are aware, the dihydrotetrabenazine isomers derived from the unstable *RS* and *SR* isomers (hereinafter referred to individually or collectively as *cis*-tetrabenazine because the hydrogen atoms at the 3 and 11b positions have a *cis* relative orientation) of tetrabenazine have not previously been isolated and characterised, and the biological activities of these compounds have not been published hitherto.

### Summary of the Invention

It has now been found that dihydrotetrabenazine isomers derived from the unstable *RS* and *SR* isomers ("cis" isomers") of tetrabenazine are not only stable but have unexpectedly good biological properties. In particular, certain of the isomers have receptor-activity profiles that are suggestive of a number of advantages over the *RR*/*SS* tetrabenazine currently in use. For example, several of the isomers, although having high affinity for VMAT2, show greatly reduced or negligible binding of dopamine receptors indicating that they are unlikely to give rise to the dopaminergic side effects encountered with tetrabenazine. None of the isomers showed inhibition of the dopamine transporter (DAT). In addition, studies in rats on several of the isomers have shown that they lack the unwanted sedative side effects associated with tetrabenazine. The lack of sedative activity may be due to the very low affinities of some of the isomers for adrenergic receptors. Furthermore, whereas one of the side effects of tetrabenazine is depression, several of the dihydrotetrabenazine isomers show an affinity for the serotonin transporter (SERT) protein indicating that they may have antidepressant activity.

Accordingly, in a first aspect, the invention provides 3,11b-*cis-*dihydrotetrabenazine.

In another aspect, the invention provides a pharmaceutical composition comprising 3,11b-*cis*-dihydrotetrabenazine and a pharmaceutically acceptable carrier.

The invention also provides 3,11b-*cis*-dihydrotetrabenazine in substantially pure form, for example at an isomeric purity of greater than 90%, typically greater than 95% and more preferably greater than 98%.

The term "isomeric purity" in the present context refers to the amount of 3,11b*-cis-*dihydrotetrabenazine present relative to the total amount or concentration of dihydrotetrabenazine of all isomeric forms. For example, if 90% of the total dihydrotetrabenazine present in the composition is 3,11b-*cis*-dihydrotetrabenazine, then the isomeric purity is 90%.

The invention further provides a composition comprising 3,11b*-cis-*dihydrotetrabenazine substantially free of 3,11b-*trans*-dihydrotetrabenazine, preferably containing less than 5% of 3,11b-*trans*-dihydrotetrabenazine, more preferably less than 3% of 3,11b-*trans*-dihydrotetrabenazine, and most preferably less than 1% of 3,11b-*trans*-dihydrotetrabenazine.

In another aspect, the invention provides 3,11b-*cis*-dihydrotetrabenazine for use in medicine or therapy, for example in the treatment of hyperkinetic movement disorders such as Huntington's disease, hemiballismus, senile chorea, tic, tardive dyskinesia and Tourette's syndrome, or in the treatment of depression.

In a further aspect, the invention provides the use of 3,11b-*cis*-dihydrotetrabenazine for the manufacture of a medicament for the treatment of hyperkinetic movement disorders such as Huntington's disease, hemiballismus, senile chorea, tic, tardive dyskinesia and Tourette's syndrome, or the treatment of depression.

The term "3,11b-*cis*-" as used herein means that the hydrogen atoms at the 3- and 11b-positions of the dihydrotetrabenazine structure are in the *cis* relative orientation. The isomers of the invention are therefore compounds of the formula (I) and antipodes (mirror images) thereof.

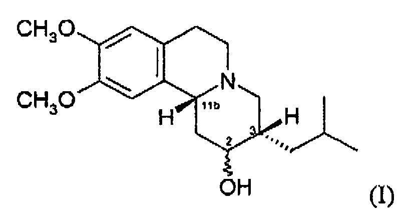

There are four possible isomers of dihydrotetrabenazine having the 3,11 b-cis configuration and these are the 2*S*,3*S*,11b*R* isomer, the 2*R*,3*R*,11b*S* isomer, the 2*R*,3*S*,11b*R* isomer and the 2*S*,3*R*,11b*S* isomer. The four isomers have been isolated and characterised and, in another aspect, the invention provides individual isomers of 3,11b-*cis*-dihydrotetrabenazine. In particular, the invention provides:
(a) the 2*S*,3*S*,11b*R* isomer of 3,11b-*cis*-dihydrotetrabenazine having the formula (Ia): 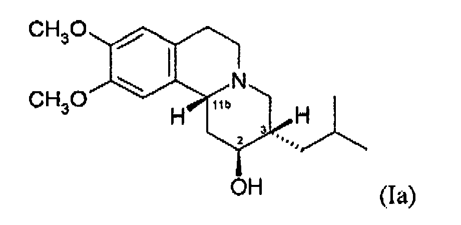
(b) the 2*R*,3*R*,11b*S* isomer of 3,11b-*cis*-dihydrotetrabenazine having the formula (Ib): 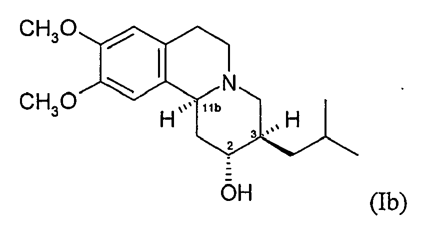
(c) the 2*R*,3*S*,11b*R* isomer of 3,11b-*cis*-dihydrotetrabenazine having the formula (Ic): 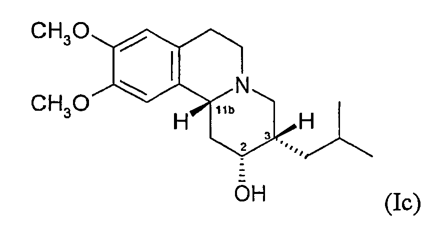 and
(d) the 2*S*,3*R*,11b*S* isomer of 3,11b-*cis*-dihydrotetrabenazine having the formula (Id):

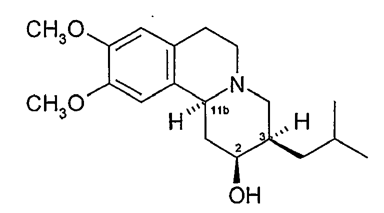

The individual novel isomers of the invention can be characterised by their spectroscopic, optical and chromatographic properties.

Preferred isomers are the dextrorotatory (+) isomers.

Without implying any particular absolute configuration or stereochemistry, the four novel isomers may be characterised as follows:

### Isomer A

Optical activity as measured by ORD (methanol, 21°C): laevorotatory (-) IR Spectrum (KBr solid), ¹H-NMR spectrum (CDCl₃) and ¹³C-NMR spectrum (CDCl₃) substantially as described in Table 1.

### Isomer B

Optical activity as measured by ORD (methanol, 21°C): dextrorotatory (+) IR Spectrum (KBr solid), ¹H-NMR spectrum (CDCl₃) and ¹³C-NMR spectrum (CDCl₃) substantially as described in Table 1.

### Isomer C

Optical activity as measured by ORD (methanol, 21°C): dextrorotatory (+) IR Spectrum (KBr solid), ¹H-NMR spectrum (CDCl₃) and ¹³C-NMR spectrum (CDCl₃) substantially as described in Table 2.

### Isomer D

Optical activity as measured by ORD (methanol, 21°C): laevorotatory (-) IR Spectrum (KBr solid), ¹H-NMR spectrum (CDCl₃) and ¹³C-NMR spectrum (CDCl₃) substantially as described in Table 2.

ORD values for each isomer are given in the examples below but it is noted that such values are given by way of example and may vary according to the degree of purity of the isomer and the influence of other variables such as temperature fluctuations and the effects of residual solvent molecules.

The enantiomers A, B, C and D may each be presented in a substantially enantiomerically pure form or as mixtures with other enantiomers of the invention.

The terms "enantiomeric purity" and "enantiomerically pure" in the present context refer to the amount of a given enantiomer of 3,11b-*cis*-dihydrotetrabenazine present relative to the total amount or concentration of dihydrotetrabenazine of all enantiomeric and isomeric forms. For example, if 90% of the total dihydrotetrabenazine present in the composition is in the form of a single enantiomer, then the enantiomeric purity is 90%.

By way of example, in each aspect and embodiment of the invention, each individual enantiomer selected from Isomers A, B, C and D may be present in an enantiomeric purity of at least 55% (e.g. at least 60%, 65%, 70%, 75%, 80%, 85%, 90%, 95%, 97%, 98%, 99%, 99.5% or 100%).

The isomers of the invention may also be presented in the form of mixtures of one or more of Isomers A, B, C and D. Such mixtures may be racemic mixtures or non-racemic mixtures. Examples of racemic mixtures include the racemic mixture of Isomer A and Isomer B and the racemic mixture of Isomer C and Isomer D.

### Pharmaceutically Acceptable Salts

Unless the context requires otherwise, a reference in this application to dihydrotetrabenazine and its isomers, includes within its scope not only the free base of the dihydrotetrabenazine but also its salts, and in particular acid addition salts.

Particular acids from which the acid addition salts are formed include acids having a pKa value of less than 3.5 and more usually less than 3. For example, the acid addition salts can be formed from an acid having a pKa in the range from +3.5 to -3.5.

Preferred acid addition salts include those formed with sulphonic acids such as methanesulphonic acid, ethanesulphonic acid, benzene sulphonic acid, toluene sulphonic acid, camphor sulphonic acid and naphthalene sulphonic acid.

One particular acid from which acid addition salts may be formed is methanesulphonic acid.

Acid addition salts can be prepared by the methods described herein or conventional chemical methods such as the methods described in Pharmaceutical Salts: Properties, Selection, and Use, P. Heinrich Stahl (Editor), Camille G. Wermuth (Editor), ISBN: 3-90639-026-8, Hardcover, 388 pages, August 2002. Generally, such salts can be prepared by reacting the free base form of the compound with the appropriate base or acid in water or in an organic solvent, or in a mixture of the two; generally, nonaqueous media such as ether, ethyl acetate, ethanol, isopropanol, or acetonitrile are used.

The salts are typically pharmaceutically acceptable salts. However, salts that are not pharmaceutically acceptable may also be prepared as intermediate forms which may then be converted into pharmaceutically acceptable salts. Such non-pharmaceutically acceptable salt forms also form part of the invention.

### Methods for the Preparation of Dihydrotetrabenazine Isomers

In a further aspect, there is provided a process (Process A) for preparing a dihydrotetrabenazine of the invention, which process comprises the reaction of a compound of the formula (II): 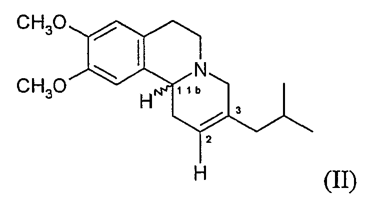 with a reagent or reagents suitable for hydrating the 2,3-double bond in the compound of formula (II) and thereafter where required separating and isolating a desired dihydrotetrabenazine isomer form .

The hydration of the 2,3-double bond can be carried out by hydroboration using a borane reagent such as diborane or a borane-ether (e.g. borane-tetrahydrofuran (THF)) to give an intermediate alkyl borane adduct followed by oxidation of the alkyl borane adduct and hydrolysis in the presence of a base. The hydroboration is typically carried out in a dry polar non-protic solvent such as an ether (e.g. THF), usually at a non-elevated temperature, for example room temperature. The borane-alkene adduct is typically oxidised with an oxidising agent such as hydrogen peroxide in the presence of a base providing a source of hydroxide ions, such as ammonium hydroxide or an alkali metal hydroxide, e.g. potassium hydroxide or sodium hydroxide. The hydroboration-oxidation-hydrolysis sequence of reactions of Process A typically provides dihydrotetrabenazine isomers in which the hydrogen atoms at the 2- and 3-positions have a *trans* relative orientation.

Compounds of the formula (II) can be prepared by reduction of tetrabenazine to give a dihydrotetrabenazine followed by dehydration of the dihydrotetrabenazine. Reduction of the tetrabenazine can be accomplished using an aluminium hydride reagent such as lithium aluminium hydride, or a borohydride reagent such as sodium borohydride, potassium borohydride or a borohydride derivative, for example an alkyl borohydride such as lithium tri-sec-butyl borohydride. Alternatively, the reduction step can be effected using catalytic hydrogenation, for example over a Raney nickel or platinum oxide catalyst. Suitable conditions for performing the reduction step are described in more detail below or can be found in US 2,843,591 (Hoffmann- La Roche) and Brossi et al., Helv. Chim. Acta., vol. XLI, No. 193, pp1793-1806 (1958).

Because the tetrabenazine used as the starting material for the reduction reaction is typically a mixture of the *RR* and *SS* isomers (i.e. *trans*-tetrabenazine), the dihydrotetrabenazine formed by the reduction step will have the same *trans* configuration about the 3- and 11b positions and will take the form of one or more of the known dihydrotetrabenazine isomers shown in Figure 3 above. Thus Process A may involve taking the known isomers of dihydrotetrabenazine, dehydrating them to form the alkene (II) and then "rehydrating" the alkene (II) using conditions that give the required novel *cis* dihydrotetrabenazine isomers of the invention.

Dehydration of the dihydrotetrabenazine to the alkene (II) can be carried out using a variety of standard conditions for dehydrating alcohols to form alkenes, see for example J. March (*idem*) pages 389-390 and references therein. Examples of such conditions include the use of phosphorus-based dehydrating agents such as phosphorus halides or phosphorus oxyhalides, e.g. POCl₃ and PCl₅. As an alternative to direct dehydration, the hydroxyl group of the dihydrotetrabenazine can be converted to a leaving group L such as halogen (e.g. chlorine or bromine) and then subjected to conditions (e.g. the presence of a base) for eliminating H-L. Conversion of the hydroxyl group to a halide can be achieved using methods well known to the skilled chemist, for example by reaction with carbon tetrachloride or carbon tetrabromide in the presence of a trialkyl or triaryl phosphine such as triphenyl phosphine or tributyl phosphine.

The tetrabenazine used as the starting material for the reduction to give the dihydrotetrabenazine can be obtained commercially or can be synthesised by the method described in US 2,830,993 (Hoffmann-La Roche).

The invention also provides a process (Process B) for preparing a dihydrotetrabenazine of the invention, which process comprises subjecting a compound of the formula (III): 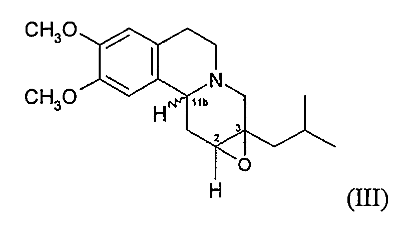 to conditions for ring-opening the 2,3-epoxide group in the compound of the formula (III), and thereafter where required separating and isolating a desired dihydrotetrabenazine isomer form.

The ring-opening can be effected in accordance with known methods for epoxide ring openings. However, a currently preferred method of ring-opening the epoxide is reductive ring opening which can be achieved using a reducing agent such as borane-THF. Reaction with borane-THF can be carried out in a polar non-protic solvent such as an ether (e.g. tetrahydrofuran) usually at ambient temperature, the borane complex thus formed being subsequently hydrolysed by heating in the presence of water and a base at the reflux temperature of the solvent. Process B typically gives rise to dihydrotetrabenazine isomers in which the hydrogen atoms at the 2- and 3-positions have a *cis* relative orientation.

The epoxide compounds of the formula (III) can be prepared by epoxidation of an alkene of the formula (II) above. The epoxidation reaction can be carried out using conditions and reagents well known to the skilled chemist, see for example J. March (*idem*), pages 826-829 and references therein. Typically, a per-acid such as *meta*-chloroperbenzoic acid (MCPBA), or a mixture of a per-acid and a further oxidising agent such as perchloric acid, may be used to bring about epoxidation.

When the starting materials for processes A and B above are mixtures of enantiomers, then the products of the processes will typically be pairs of enantiomers, for example racemic mixtures, possibly together with diastereoisomeric impurities. Unwanted diastereoisomers can be removed by techniques such as chromatography (e.g. HPLC) and the individual enantiomers can be separated by a variety of methods known to the skilled chemist. For example, they can be separated by means of:
(i) chiral chromatography (chromatography on a chiral support); or
(ii) forming a salt with an optically pure chiral acid, separating the salts of the two diastereoisomers by fractional crystallisation and then releasing the dihydrotetrabenazine from the salt; or
(iii) forming a derivative (such as an ester) with an optically pure chiral derivatising agent (e.g. esterifying agent), separating the resulting epimers (e.g. by chromatography) and then converting the derivative to the dihydrotetrabenazine.

One method of separating pairs of enantiomers obtained from each of Processes A and B, and which has been found to be particularly effective, is to esterify the hydroxyl group of the dihydrotetrabenazine with an optically active form of Mosher's acid, such as the *R* (+) isomer shown below, or an active form thereof:

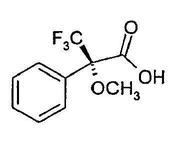

The resulting esters of the two enantiomers of the dihydrobenazine can then be separated by chromatography (e.g. HPLC) and the separated esters hydrolysed to give the individual dihydrobenazine isomers using a base such as an alkali metal hydroxide (e.g. NaOH) in a polar solvent such as methanol.

As an alternative to using mixtures of enantiomers as the starting materials in processes A and B and then carrying out separation of enantiomers subsequently, processes A and B can each be carried out on single enantiomer starting materials leading to products in which a single enantiomer predominates. Single enantiomers of the alkene (II) can be prepared by subjecting RR/SS tetrabenazine to a stereoselective reduction using lithium tri-sec-butyl borohydride to give a mixture of SRR and RSS enantiomers of dihydrotetrabenazine, separating the enantiomers (e.g. by fractional crystallisation) and then dehydrating a separated single enantiomer of dihydrotetrabenazine to give predominantly or exclusively a single enantiomer of the compound of formula (II).

Processes A and B are illustrated in more detail below in Schemes 1 and 2 respectively.

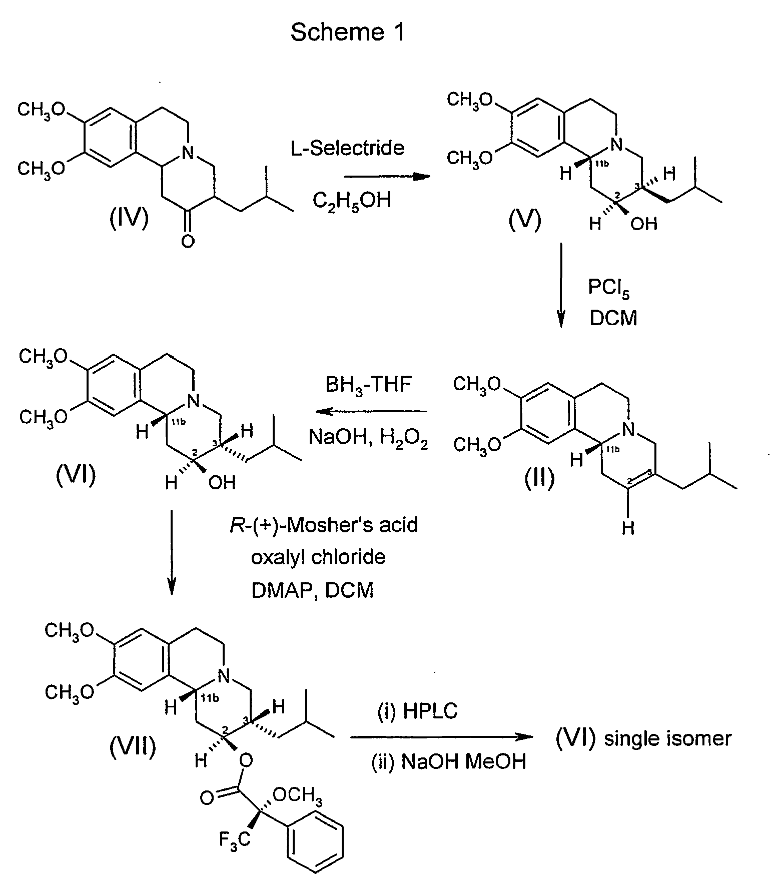

Scheme 1 illustrates the preparation of individual dihydrotetrabenazine isomers having the 2*S,*3*S,*11b*R* and 2*R*,3*R*,11b*S* configurations in which the hydrogen atoms attached to the 2- and 3-positions are arranged in a *trans* relative orientation. This reaction scheme includes Process A defined above.

The starting point for the sequence of reactions in Scheme 1 is commercially available tetrabenazine (IV) which is a racemic mixture of the RR and SS optical isomers of tetrabenazine. In each of the RR and SS isomers, the hydrogen atoms at the 3- and 11b-positions are arranged in a *trans* relative orientation. As an alternative to using the commercially available compound, tetrabenazine can be synthesised according to the procedure described in US patent number 2,830,993 (see in particular example 11).

The racemic mixture of RR and SS tetrabenazine is reduced using the borohydride reducing agent lithium tri-sec-butyl borohydride ("L-Selectride") to give a mixture of the known 2*S*,3*R*,11b*R* and 2*R*,3*S*,11b*S* isomers (V) of dihydrotetrabenazine, of which only the 2*S*,3*R*,11b*R* isomer is shown for simplicity. By using the more sterically demanding L-Selectride as the borohydride reducing agent rather than sodium borohydride, formation of the RRR and SSS isomers of dihydrotetrabenazine is minimised or suppressed.

The dihydrotetrabenazine isomers (V) are reacted with a dehydrating agent such as phosphorus pentachloride in a non-protic solvent such as a chlorinated hydrocarbon (for example chloroform or dichloromethane, preferably dichloromethane) to form the unsaturated compound (II) as a pair of enantiomers, only the R-enantiomer of which is shown in the Scheme. The dehydration reaction is typically carried out at a temperature lower than room temperature, for example at around 0-5°C.

The unsaturated compound (II) is then subjected to a stereoselective re-hydration to generate the dihydrotetrabenazine (VI) and its mirror image or antipode (not shown) in which the hydrogen atoms at the 3- and 11b-positions are arranged in a *cis* relative orientation and the hydrogen atoms at the 2- and 3-positions are arranged in a *trans* relative orientation. The stereoselective rehydration is accomplished by a hydroboration procedure using borane-THF in tetrahydrofuran (THF) to form an intermediate borane complex (not shown) which is then oxidised with hydrogen peroxide in the presence of a base such as sodium hydroxide.

An initial purification step may then be carried out (e.g. by HPLC) to give the product (V) of the rehydration reaction sequence as a mixture of the 2*S*,3*S*,11b*R* and 2*R*,3*R*,11b*S* isomers of which only the 2*S*,3*S*,11b*R* isomer is shown in the Scheme. In order to separate the isomers, the mixture is treated with R (+) Mosher's acid, in the presence of oxalyl chloride and dimethylaminopyridine (DMAP) in dichloromethane to give a pair of diastereoisomeric esters (VII) (of which only one diastereoisomer is shown) which can then be separated using HPLC. The individual esters can then be hydrolysed using an alkali metal hydroxide such as sodium hydroxide to give a single isomer (VI).

In a variation of the sequence of steps shown in Scheme 1, following the reduction of RR/SS tetrabenazine, the resulting mixture of enantiomers of the dihydrotetrabenazine (V) can be separated to give the individual enantiomers. Separation can be carried out by forming a salt with a chiral acid such as (+) or (-) camphorsulphonic acid, separating the resulting diastereoisomers by fractional crystallisation to give a salt of a single enantiomer and then releasing the free base from the salt.

The separated dihydrotetrabenazine enantiomer can be dehydrated to give a single enantiomer of the alkene (II). Subsequent rehydration of the alkene (II) will then give predominantly or exclusively a single enantiomer of the cis-dihydrotetrabenazine (VI). An advantage of this variation is that it does not involve the formation of Mosher's acid esters and therefore avoids the chromatographic separation typically used to separate Mosher's acid esters.

Scheme 2 illustrates the preparation of individual dihydrotetrabenazine isomers having the 2*R*,3*S*,11b*R* and 2*S*,3*R*,11b*S* configurations in which the hydrogen atoms attached to the 2- and 3-positions are arranged in a *cis* relative orientation. This reaction scheme includes Process B defined above.

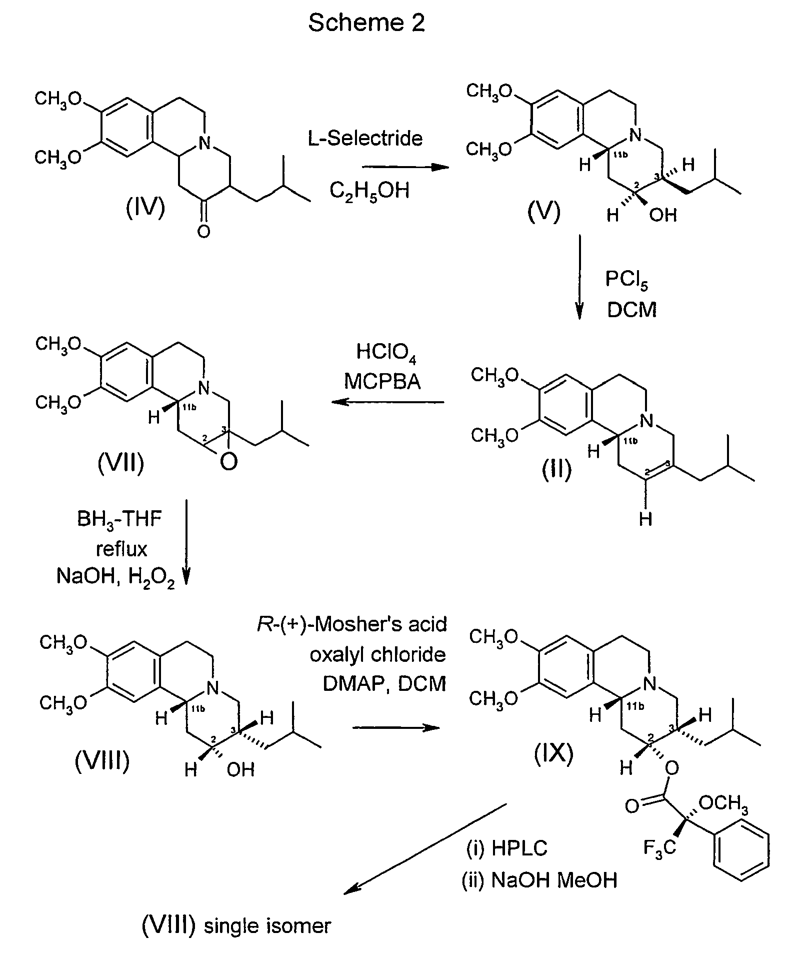

In Scheme 2, the unsaturated compound (II) is produced by reducing tetrabenazine to give the 2S,3R,11bR and 2R,3S,11bS isomers (V) of dihydrotetrabenazine and dehydrating with PCl₅ in the manner described above in Scheme 1. However, instead of subjecting the compound (II) to hydroboration, the 2,3-double bond is converted to an epoxide by reaction with *meta*-chloroperbenzoic acid (MCPBA) and perchloric acid. The epoxidation reaction is conveniently carried out in an alcohol solvent such as methanol, typically at around room temperature.

The epoxide (VII) is then subjected to a reductive ring opening using borane-THF as an electrophilic reducing agent to give an intermediate borane complex (not shown) which is then oxidised and cleaved with hydrogen peroxide in the presence of an alkali such as sodium hydroxide to give a dihydrotetrabenazine (VIII) as a mixture of the 2*R*,3*S*,11b*R* and 2*S*,3*R*,11b*S* isomers, of which only the 2*R*,3*S*,11b*R* is shown for simplicity. Treatment of the mixture of isomers (VIII) with R (+) Mosher's acid in the presence of oxalyl chloride and dimethylaminopyridine (DMAP) in dichloromethane gives a pair of epimeric esters (IX) (of which only one epimer is shown) which can then by separated by chromatography and hydrolysed with sodium hydroxide in methanol in the manner described above in relation to Scheme 1.

The chemical intermediates (II) and (III) are believed to be new and represent a further aspect of the invention.

### Biological Properties and Therapeutic Uses

Tetrabenazine exerts its therapeutic effects by inhibiting the vesicular monoamine transporter VMAT2 in the brain and by inhibiting both pre-synaptic and postsynaptic dopamine receptors.

The novel dihydrotetrabenazine isomers of the invention are also inhibitors of VMAT2, with Isomers C and B producing the greatest degree of inhibition. Like tetrabenazine, the compounds of the invention have only a low affinity for VMAT1, the VMAT isoform found in peripheral tissues and some endocrine cells, thereby indicating that they should not produce the side effects associated with reserpine. Compounds C and B also exhibit no inhibitory activity against catechol O-methyl transferase (COMT), monoamine oxidase isoforms A and B, and 5-hydroxytryptamine isoforms 1d and 1b.

Surprisingly, isomers C and B also show a remarkable separation of VAMT2 and dopamine receptor activity in that although they are highly active in binding VMAT2, both compounds exhibit only weak or non-existent dopamine receptor binding activity and lack Dopamine Transporter (DAT) binding activity. In fact, none of the isomers exhibit significant DAT binding activity. This suggests that the compounds may lack the dopaminergic side effects produced by tetrabenazine. Isomers C and B are also either weakly active or inactive as inhibitors of the adrenergic receptors and this suggests that the compounds may lack the adrenergic side effects often encountered with tetrabenazine. In fact, in locomotor studies carried out on rats, tetrabenazine exhibited a dose related sedative effect, whereas no sedative effects were observed following administration of the dihydrotetrabenazine isomers B and C of the invention.

Furthermore, both Isomer C and Isomer B are potent inhibitors of the serotonin transporter protein SERT. Inhibition of SERT is one mechanism by which antidepressants such as fluoxetine (Prozac^{®}) exert their therapeutic effects. Therefore, the ability of Isomers C and B to inhibit SERT indicates that these isomers may act as antidepressants, in marked contrast to tetrabenazine for which depression is a well recognised side effect.

On the basis of the studies carried out to date, it is envisaged that the dihydrotetrabenazine compounds of the invention will be useful in the prophylaxis or treatment of the disease states and conditions for which tetrabenazine is currently used or proposed. Thus, by way of example, and without limitation, the dihydrotetrabenazine compounds of the invention may be used for the treatment of hyperkinetic movement disorders such as Huntington's disease, hemiballismus, senile chorea, tic disorders, tardive dyskinesia, dystonia and Tourette's syndrome.

It is also envisaged that the dihydrotetrabenazine compounds of the invention may be useful in the treatment of depression.

The compounds will generally be administered to a subject in need of such administration, for example a human or animal patient, preferably a human.

The compounds will typically be administered in amounts that are therapeutically or prophylactically useful and which generally are non-toxic. However, in certain situations, the benefits of administering a dihydrotetrabenazine compound of the invention may outweigh the disadvantages of any toxic effects or side effects, in which case it may be considered desirable to administer compounds in amounts that are associated with a degree of toxicity.

A typical daily dose of the compound can be in the range from 0.025 milligrams to 5 milligrams per kilogram of body weight, for example up to 3 milligrams per kilogram of bodyweight, and more typically 0.15 milligrams to 5 milligrams per kilogram of bodyweight although higher or lower doses may be administered where required.

By way of example, an initial starting dose of 12.5 mg may be administered 2 to 3 times a day. The dosage can be increased by 12.5 mg a day every 3 to 5 days until the maximal tolerated and effective dose is reached for the individual as determined by the physician. Ultimately, the quantity of compound administered will be commensurate with the nature of the disease or physiological condition being treated and the therapeutic benefits and the presence or absence of side effects produced by a given dosage regimen, and will be at the discretion of the physician.

### Pharmaceutical Formulations

The invention also provides dihydrotetrabenazine compounds as hereinbefore defined in the form of pharmaceutical compositions.

The pharmaceutical compositions can be in any form suitable for oral, parenteral, topical, intranasal, intrabronchial, ophthalmic, otic, rectal, intra-vaginal, or transdermal administration. Where the compositions are intended for parenteral administration, they can be formulated for intravenous, intramuscular, intraperitoneal, subcutaneous administration or for direct delivery into a target organ or tissue by injection, infusion or other means of delivery.

Pharmaceutical dosage forms suitable for oral administration include tablets, capsules, caplets, pills, lozenges, syrups, solutions, sprays, powders, granules, elixirs and suspensions, sublingual tablets, sprays, wafers or patches and buccal patches.

Pharmaceutical compositions containing the dihydrotetrabenazine compounds of the invention can be formulated in accordance with known techniques, see for example, Remington's Pharmaceutical Sciences, Mack Publishing Company, Easton, PA, USA.

Thus, tablet compositions can contain a unit dosage of active compound together with an inert diluent or carrier such as a sugar or sugar alcohol, e.g.; lactose, sucrose, sorbitol or mannitol; and/or a non-sugar derived diluent such as sodium carbonate, calcium phosphate, talc, calcium carbonate, or a cellulose or derivative thereof such as methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Capsule formulations may be of the hard gelatin or soft gelatin variety and can contain the active component in solid, semi-solid, or liquid form. Gelatin capsules can be formed from animal gelatin or synthetic or plant derived equivalents thereof.

The solid dosage forms (e.g.; tablets, capsules etc.) can be coated or un-coated, but typically have a coating, for example a protective film coating (e.g. a wax or varnish) or a release controlling coating. The coating (e.g. a Eudragit ^{™} type polymer) can be designed to release the active component at a desired location within the gastro-intestinal tract. Thus, the coating can be selected so as to degrade under certain pH conditions within the gastrointestinal tract, thereby selectively release the compound in the stomach or in the ileum or duodenum.

Instead of, or in addition to, a coating, the drug can be presented in a solid matrix comprising a release controlling agent, for example a release delaying agent which may be adapted to selectively release the compound under conditions of varying acidity or alkalinity in the gastrointestinal tract. Alternatively, the matrix material or release retarding coating can take the form of an erodible polymer (e.g. a maleic anhydride polymer) which is substantially continuously eroded as the dosage form passes through the gastrointestinal tract.

Compositions for topical use include ointments, creams, sprays, patches, gels, liquid drops and inserts (for example intraocular inserts). Such compositions can be formulated in accordance with known methods.

Compositions for parenteral administration are typically presented as sterile aqueous or oily solutions or fine suspensions, or may be provided in finely divided sterile powder form for making up extemporaneously with sterile water for injection.

Examples of formulations for rectal or intra-vaginal administration include pessaries and suppositories which may be, for example, formed from a shaped mouldable or waxy material containing the active compound.

Compositions for administration by inhalation may take the form of inhalable powder compositions or liquid or powder sprays, and can be administrated in standard form using powder inhaler devices or aerosol dispensing devices. Such devices are well known. For administration by inhalation, the powdered formulations typically comprise the active compound together with an inert solid powdered diluent such as lactose.

The compounds of the inventions will generally be presented in unit dosage form and, as such, will typically contain sufficient compound to provide a desired level of biological activity. For example, a formulation intended for oral administration may contain from 2 milligrams to 200 milligrams of active ingredient, more usually from 10 milligrams to 100 milligrams, for example, 12.5 milligrams, 25 milligrams and 50 milligrams.

The active compound will be administered to a patient in need thereof (for example a human or animal patient) in an amount sufficient to achieve the desired therapeutic effect.

### EXAMPLES

The following examples illustrate the synthesis and properties of the dihydrotetrabenazine compounds of the invention.

### EXAMPLE 1

### Preparation of 2S,3S,11bR and 2R,3R,11bS Isomers of Dihydrotetrabenazine

### 1A. Reduction of RR/SS Tetrabenazine

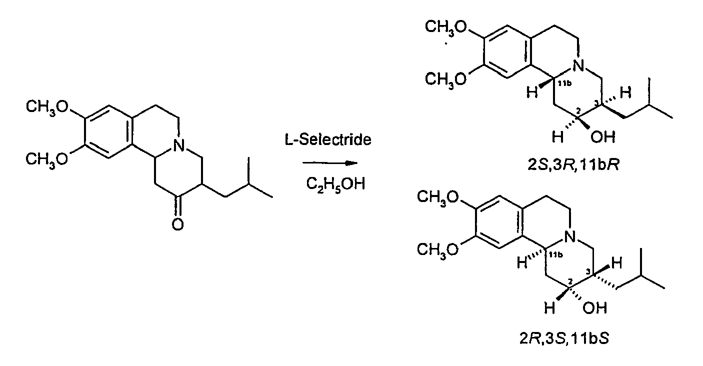

1M L-Selectride^{®} in tetrahydrofuran (135 ml, 135 mmol, 2.87 eq.) was added slowly over 30 minutes to a stirred solution of tetrabenazine *RR*/*SS* racemate (15 g, 47 mmol) in ethanol (75 ml) and tetrahydrofuran (75 ml) at 0 °C. After addition was complete the mixture was stirred at 0 °C for 30 minutes and then allowed to warm to room temperature.

The mixture was poured onto crushed ice (300 g) and water (100 ml) added. The solution was extracted with diethyl ether (2 x 200 ml) and the combined ethereal extracts washed with water (100 ml) and partly dried over anhydrous potassium carbonate. Drying was completed using anhydrous magnesium sulphate and, after filtration, the solvent was removed at reduced pressure (shielded from the light, bath temperature <20 °C) to afford a pale yellow solid.

The solid was slurried with petroleum ether (30-40 °C) and filtered to afford a white powdery solid (12 g, 80%).

### 1B. Dehydration of reduced Tetrabenazine

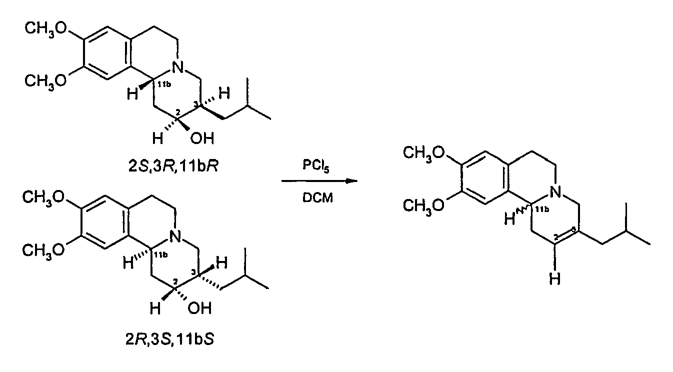

Phosphorous pentachloride (32.8 g, 157.5 mmol, 2.5 eq) was added in portions over 30 minutes to a stirred solution of the reduced tetrabenazine product from Example 1A (20 g, 62.7 mmol) in dichloromethane (200 ml) at 0 °C. After the addition was complete, the reaction mixture was stirred at 0 °C for a further 30 minutes and the solution poured slowly into 2M aqueous sodium carbonate solution containing crushed ice (0 °C). Once the initial acid gas evolution had ceased the mixture was basified (ca. pH 12) using solid sodium carbonate.

The alkaline solution was extracted using ethyl acetate (800 ml) and the combined organic extracts dried over anhydrous magnesium sulphate. After filtration the solvent was removed at reduced pressure to afford a brown oil, which was purified by column chromatography (silica, ethyl acetate) to afford the semi-pure alkene as a yellow solid (10.87 g, 58%).

### 1C. Hydration of the Crude Alkene from Example 1B

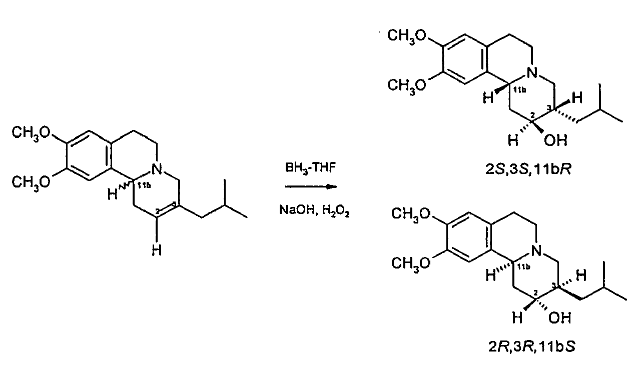

A solution of the crude alkene (10.87 g, 36.11 mmol) from Example 1B in dry THF (52 ml) at room temperature was treated with 1M borane-THF (155.6 ml, 155.6 mmol, 4.30 eq) added in a dropwise manner. The reaction was stirred for 2 hours, water (20 ml) was added and the solution basified to pH 12 with 30% aqueous sodium hydroxide solution.

Aqueous 30% hydrogen peroxide solution (30 ml) was added to the stirred alkaline reaction mixture and the solution was heated to reflux for 1 hour before being allowed to cool. Water (100 ml) was added and the mixture extracted with ethyl acetate (3 x 250 ml). The organic extracts were combined and dried over anhydrous magnesium sulphate and after filtration the solvent was removed at reduced pressure to afford a yellow oil (9 g).

The oil was purified using preparative HPLC (Column: Lichrospher Si60, 5 µm, 250 x 21.20 mm, mobile phase: hexane : ethanol : dichloromethane (85:15:5); UV 254 nm, flow: 10 ml min⁻¹) at 350 mg per injection followed by concentration of the fractions of interest under vacuum. The product oil was then dissolved in ether and concentrated once more under vacuum to give the dihydrotetrabenazine racemate shown above as a yellow foam (5.76 g, 50%).

### 1D. Preparation of Mosher's ester derivatives

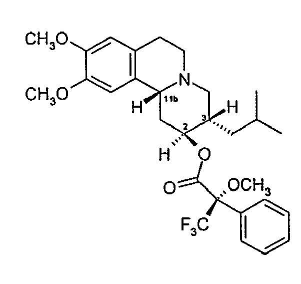

and

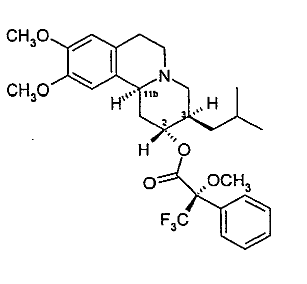

R-(+)-α-methoxy-α-trifluoromethylphenyl acetic acid (5 g, 21.35 mmol), oxalyl chloride (2.02 ml) and DMF (0.16 ml) were added to anhydrous dichloromethane (50 ml) and the solution was stirred at room temperature for 45 minutes. The solution was concentrated under reduced pressure and the residue was taken up in anhydrous dichloromethane (50 ml) once more. The resulting solution was cooled using an ice-water bath and dimethylaminopyridine (3.83 g, 31.34 mmol) was added followed by a pre-dried solution (over 4Å sieves) in anhydrous dichloromethane of the solid product of Example 1C (5 g, 15.6 mmol). After stirring at room temperature for 45 minutes, water (234 ml) was added and the mixture extracted with ether (2 x 200 ml). The ether extract was dried over anhydrous magnesium sulphate, passed through a pad of silica and the product eluted using ether.

The collected ether eluate was concentrated under reduced pressure to afford an oil which was purified using column chromatography (silica, hexane : ether (10:1)). Evaporation of the collected column fractions of interest and removal of the solvent at reduced pressure gave a solid which was further purified using column chromatography (silica, hexane : ethyl acetate (1:1)) to give three main components which were partially resolved into Mosher's ester peaks 1 and 2.

Preparative HPLC of the three components (Column: 2 x Lichrospher Si60, 5 µm, 250 x 21.20 mm, mobile phase: hexane : isopropanol (97:3), UV 254 nm; flow: 10 ml min⁻¹) at 300 mg loading followed by concentration of the fractions of interest under vacuum gave the pure Mosher's ester derivatives
Peak 1 (3.89 g, 46.5%)
Peak 2 (2.78 g, 33%)

The fractions corresponding to the two peaks were subjected to hydrolysis to liberate the individual dihydrotetrabenazine isomers identified and characterised as Isomers A and B. Isomers A and B are each believed to have one of the following structures

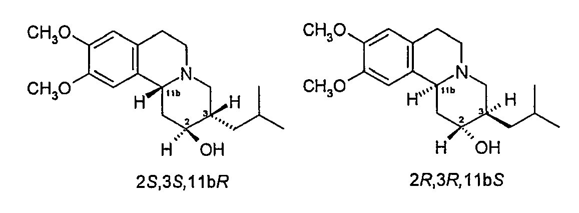

### 1E. Hydrolysis of Peak 1 to give Isomer A

Aqueous 20% sodium hydroxide solution (87.5 ml) was added to a solution of Mosher's ester peak 1 (3.89 g, 7.27 mmol) in methanol (260 ml) and the mixture stirred and heated to reflux for 150 minutes. After cooling to room temperature water (200 ml) was added and the solution extracted with ether (600 ml), dried over anhydrous magnesium sulphate and after filtration, concentrated under reduced pressure.

The residue was dissolved using ethyl acetate (200 ml), the solution washed with water (2 x 50 ml), the organic phase dried over anhydrous magnesium sulphate and after filtration, concentrated under reduced pressure to give a yellow foam. This material was purified by column chromatography (silica, gradient elution of ethyl acetate : hexane (1:1) to ethyl acetate). The fractions of interest were combined and the solvent removed at reduced pressure. The residue was taken up in ether and the solvent removed at reduced pressure once more to give Isomer A as an off-white foam (1.1 g, 47%).

Isomer A, which is believed to have either the 2*S,*3*S*11b*R* or 2*R*,3*R*,11b*S* configuration (the absolute stereochemistry was not determined), was characterized by ¹H-NMR, ¹³C-NMR, IR, mass spectrometry, chiral HPLC and ORD. The IR, NMR and MS data for isomer A are set out in Table 1 and the Chiral HPLC and ORD data are set out in Table 3.

### 1F. Hydrolysis of Peak 2 to give Isomer B

Aqueous 20% sodium hydroxide solution (62.5 ml) was added to a solution of Mosher's ester peak 2 (2.78 g, 5.19 mmol) in methanol (185 ml) and the mixture stirred and heated to reflux for 150 minutes. After cooling to room temperature water (142 ml) was added and the solution extracted with ether (440 ml), dried over anhydrous magnesium sulphate and after filtration, concentrated under reduced pressure.

The residue was dissolved using ethyl acetate (200 ml), the solution washed with water (2 x 50 ml), the organic phase dried over anhydrous magnesium sulphate and after filtration, concentrated under reduced pressure. Petroleum ether (30-40 °C) was added to the residue and the solution concentrated under vacuum once more to give Isomer B as a white foam (1.34 g, 81%).

Isomer B, which is believed to have either the 2*S*,3*S*,11b*R* or 2*R*,3*R*,11b*S* configuration (the absolute stereochemistry was not determined), was characterized by ¹H-NMR, ¹³C-NMR, IR, mass spectrometry, chiral HPLC and ORD. The IR, NMR and MS data for Isomer B are set out in Table 1 and the Chiral HPLC and ORD data are set out in Table 3.

### EXAMPLE 2

### Preparation of 2R,3S,11bR and 2S,3R,11bS Isomers of Dihydrotetrabenazine

### 2A. Preparation of 2,3-Dehydrotetrabenazine

A solution containing a racemic mixture (15 g, 47 mmol) of RR and SS tetrabenazine enantiomers in tetrahydrofuran was subjected to reduction with L-Selectride^{®} by the method of Example 1A to give a mixture of the 2*S*,3*R*,11b*R* and 2*R*,3*S*,11b*S* enantiomers of dihydrotetrabenazine as a white powdery solid (12 g, 80%). The partially purified dihydrotetrabenazine was then dehydrated using PCl₅ according to the method of Example 1B to give a semi-pure mixture of 11b*R* and 11b*S* isomers of 2,3-dehydrotetrabenazine (the 11b*R* enantiomer of which is shown below) as a yellow solid (12.92 g, 68%).

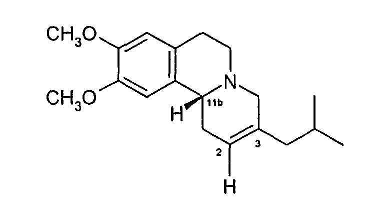

### 2B. Epoxidation of the Crude Alkene from Example 2A

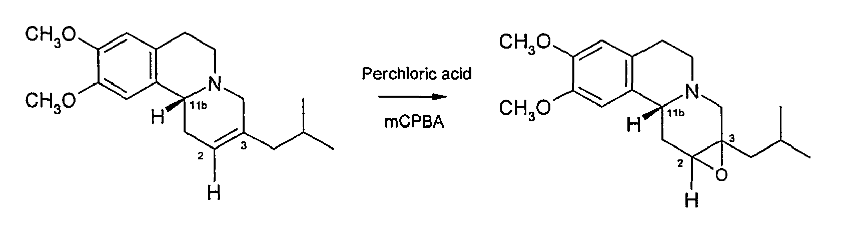

To a stirred solution of the crude alkene from Example 2A (12,92 g, 42.9 mmol) in methanol (215 ml) was added a solution of 70% perchloric acid (3.70 ml, 43 mmol) in methanol (215 ml). 77% 3-Chloroperoxybenzoic acid (15.50 g, 65 mmol) was added to the reaction and the resulting mixture was stirred for 18 hours at room temperature protected from light.

The reaction mixture was poured into saturated aqueous sodium sulphite solution (200 ml) and water (200 ml) added. Chloroform (300 ml) was added to the resulting emulsion and the mixture basified with saturated aqueous sodium bicarbonate (400 ml).

The organic layer was collected and the aqueous phase washed with additional chloroform (2 x 150 ml). The combined chloroform layers were dried over anhydrous magnesium sulphate and after filtration the solvent was removed at reduced pressure to give a brown oil (14.35 g, yield > 100% - probable solvent remains in product). This material was used without further purification.

### 2C. Reductive Ring Opening of the Epoxide from 2B

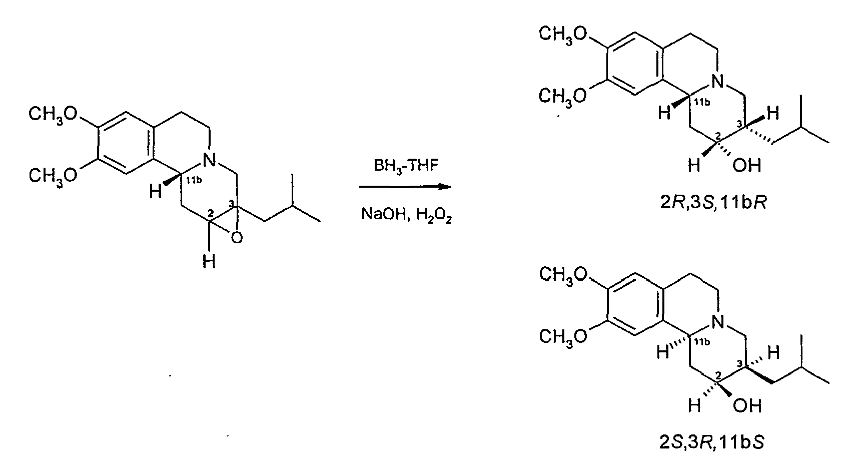

A stirred solution of the crude epoxide from Example 2B (14.35 g, 42.9 mmol, assuming 100% yield) in dry THF (80 ml) was treated slowly with 1M borane/THF (184.6 ml, 184.6 mmol) over 15 minutes. The reaction was stirred for two hours, water (65 ml) was added and the solution heated with stirring to reflux for 30 minutes.

After cooling, 30% sodium hydroxide solution (97 ml) was added to the reaction mixture followed by 30% hydrogen peroxide solution (48.6 ml) and the reaction was stirred and heated to reflux for an additional 1 hour.

The cooled reaction mixture was extracted with ethyl acetate (500 ml) dried over anhydrous magnesium sulphate and after filtration the solvent was removed at reduced pressure to give an oil. Hexane (230 ml) was added to the oil and the solution re-concentrated under reduced pressure.

The oily residue was purified by column chromatography (silica, ethyl acetate). The fractions of interest were combined and the solvent removed under reduced pressure. The residue was purified once more using column chromatography (silica, gradient, hexane to ether). The fractions of interest were combined and the solvents evaporated at reduced pressure to give a pale yellow solid (5.18 g, 38%).

### 2D. Preparation of Mosher's ester derivatives of the 2R,3S,11bR and 2S,3R,11bS Isomers of Dihydrotetrabenazine

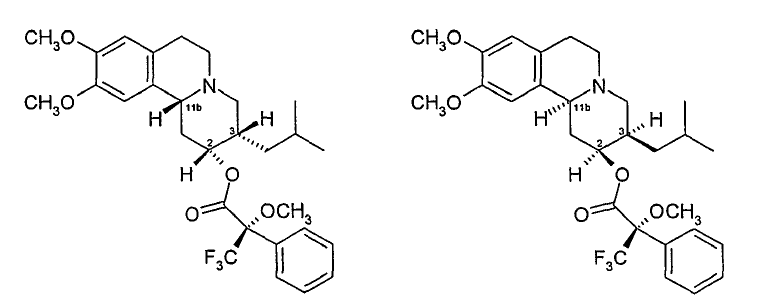

R-(+)-α-methoxy-α-trifluoromethylphenyl acetic acid (4.68 g, 19.98 mmol), oxalyl chloride (1.90 ml) and DMF (0.13 ml) were added to anhydrous dichloromethane (46 ml) and the solution stirred at room temperature for 45 minutes. The solution was concentrated under reduced pressure and the residue was taken up in anhydrous dichloromethane (40 ml) once more. The resulting solution was cooled using an ice-water bath and dimethylaminopyridine (3.65 g, 29.87 mmol) was added followed by a pre-dried solution (over 4Å sieves) in anhydrous dichloromethane (20 ml) of the solid product of Example 2C (4.68 g, 14.6 mmol). After stirring at room temperature for 45 minutes, water (234 ml) was added and the mixture extracted with ether (2 x 200 ml). The ether extract was dried over anhydrous magnesium sulphate, passed through a pad of silica and the product eluted using ether.

The collected ether eluate was concentrated under reduced pressure to afford an oil which was purified using column chromatography (silica, hexane : ether (1:1)). Evaporation of the collected column fractions of interest and removal of the solvent at reduced pressure gave a pink solid (6.53 g)

Preparative HPLC of the solid (Column: 2 x Lichrospher Si60, 5 µm, 250 x 21.20 mm; mobile phase hexane : isopropanol (97:3); UV 254 nm; flow: 10 ml min⁻¹) at 100 mg loading followed by concentration of the fractions of interest under vacuum gave a solid which was slurried with petroleum ether (30-40 °C) and collected by filtration to give the pure Mosher's ester derivatives
Peak 1 (2.37 g, 30%)
Peak 2 (2.42 g, 30%)

The fractions corresponding to the two peaks were subjected to hydrolysis to liberate the individual dihydrotetrabenazine isomers identified and characterised as Isomers C and D. Isomers C and D are each believed to have one of the following structures

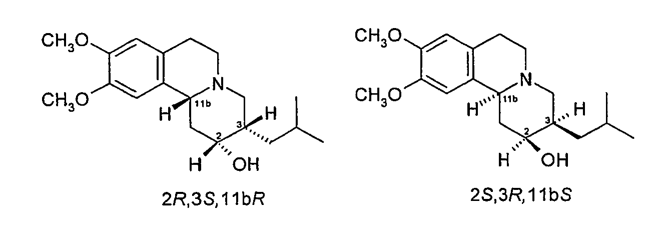

### 2F. Hydrolysis of Peak 1 to give Isomer C

20% aqueous sodium hydroxide solution (53 ml) was added to a stirred solution of Mosher's ester peak 1 (2.37 g, 4.43 mmol) in methanol (158 ml) and the mixture stirred at reflux for 150 minutes. After cooling water (88 ml) was added to the reaction mixture and the resulting solution extracted with ether (576 ml). The organic extract was dried over anhydrous magnesium sulphate and after filtration the solvent removed at reduced pressure. Ethyl acetate (200 ml) was added to the residue and the solution washed with water (2 x 50 ml). The organic solution was dried over anhydrous magnesium sulphate and after filtration the solvent removed at reduced pressure.

This residue was treated with petroleum ether (30-40 °C) and the resulting suspended solid collected by filtration. The filtrate was concentrated at reduced pressure and the second batch of suspended solid was collected by filtration. Both collected solids were combined and dried under reduced pressure to give Isomer C (1.0 g, 70%).

Isomer C, which is believed to have either the 2*R*,3*S*,11b*R* or 2*S*,3*R*,11b*S* configuration (the absolute stereochemistry was not determined), was characterized by ¹H-NMR, ¹³C-NMR, IR, mass spectrometry, chiral HPLC and ORD. The IR, NMR and MS data for Isomer C are set out in Table 2 and the Chiral HPLC and ORD data are set out in Table 4.

### 2G. Hydrolysis of Peak 2 to give Isomer D

20% aqueous sodium hydroxide solution (53 ml) was added to a stirred solution of Mosher's ester peak 2 (2.42 g, 4.52 mmol) in methanol (158 ml) and the mixture stirred at reflux for 150 minutes. After cooling water (88 ml) was added to the reaction mixture and the resulting solution extracted with ether (576 ml). The organic extract was dried over anhydrous magnesium sulphate and after filtration the solvent removed at reduced pressure. Ethyl acetate (200 ml) was added to the residue and the solution washed with water (2 x 50 ml). The organic solution was dried over anhydrous magnesium sulphate and after filtration the solvent removed at reduced pressure.

This residue was treated with petroleum ether (30-40 °C) and the resulting suspended orange solid collected by filtration. The solid was dissolved in ethyl acetate : hexane (15:85) and purified by column chromatography (silica, gradient ethyl acetate : hexane (15:85) to ethyl acetate). The fractions of interest were combined and the solvent removed at reduced pressure. The residue was slurried with petroleum ether (30-40 °C) and the resulting suspension collected by filtration. The collected solid was dried under reduced pressure to give Isomer D as a white solid (0.93 g, 64%).

Isomer D, which is believed to have either the 2*R*,3*S*,11b*R* or 2*S*,3*R*,11b*S* configuration (the absolute stereochemistry was not determined), was characterized by ¹H-NMR, ¹³C-NMR, IR, mass spectrometry, chiral HPLC and ORD. The IR, NMR and MS data for Isomer D are set out in Table 2 and the Chiral HPLC and ORD data are set out in Table 4.

In Tables 1 and 2, the infra red spectra were determined using the KBr disc method. The ¹H NMR spectra were carried out on solutions in deuterated chloroform using a Varian Gemini NMR spectrometer (200 MHz.). The ¹³C NMR spectra were carried out on solutions in deuterated chloroform using a Varian Gemini NMR spectrometer (50MHz). The mass spectra were obtained using a Micromass Platform II (ES⁺ conditions) spectrometer. In Tables 3 and 4, the Optical Rotatory Dispersion figures were obtained using an Optical Activity PolAAr 2001 instrument in methanol solution at 24°C. The HPLC retention time measurements were carried out using an HP1050 HPLC chromatograph with UV detection.

### Tables 1 and 2 - Spectroscopic Data

| Table 1 | | | | |
|---|---|---|---|---|
| Dihydrotetrabenazine isomer | ¹H-NMR spectrum (CDCl₃) | ¹³C-NMR spectrum (CDCl₃) | IR Spectrum (KBr solid) | Mass Spectrum (ES⁺) |
| Isomers A and B | 6.67δ 1H (s); | 147.7δ; | 2950 cm⁻¹; | MH⁺ 320 |
| 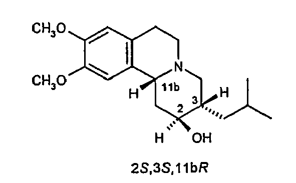 OR 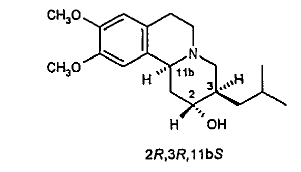 | 6.57 δ 1H (s); | 147.6δ; | 2928 cm⁻¹; | |
| | 3.84 δ 6H (s); | 130.5δ; | 2868 cm⁻¹; | |
| | 3.55 δ 1H (br. d); | 127.6δ; | 2834 cm⁻¹; | |
| | 3.08 δ 1H (m); | 112.1δ; | 1610 cm⁻¹; | |
| | 2.79 δ 2H (m); | 108.4δ; | 1511 cm⁻¹; | |
| | 2.55 δ 3H (m); | 70.5δ; | 1464 cm⁻¹ | |
| | 2.17 δ 1H (m); | 57.5 δ; | 1364 cm⁻¹; | |
| | 1.72 δ 6H (m); | 56.5 δ; | 1324 cm⁻¹; | |
| | 1.02 δ 1H (m); | 56.3 δ; | 1258 cm⁻¹; | |
| | 0.88 δ 6H (t) | 54.8 δ; | 1223 cm⁻¹; | |
| | | 53.2 δ; | 1208 cm⁻¹; | |
| | | 40.4 δ; | 1144 cm⁻¹; | |
| | | 40.1 δ; | 1045 cm⁻¹; | |
| | | 36.0 δ; | 1006 cm⁻¹; | |
| | | 28.8 δ; | 870 cm⁻¹; | |
| | | 26.2 δ; | 785 cm⁻¹; | |
| | | 23.7δ; | 764 cm⁻¹ | |
| | | 22.9 δ | | |

| Table 2 | | | | |
|---|---|---|---|---|
| Dihydrotetrabenazine isomer | ¹H-NMR spectrum (CDCl₃) | ¹³C-NMR spectrum (CDCl₃) | IR Spectrum (KBr solid) | Mass Spectrum (ES⁺) |
| Isomers C and D | 6.68 δ 1H (s); | 147.8 δ; | 3370 cm⁻¹; | MH⁺ 320 |
| | 6.58 δ 1H (s); | 147.7 δ; | 2950 cm⁻¹; | |
| | 3.92 δ 1H (m); | 130.4 δ; | 2929 cm⁻¹; | |
| 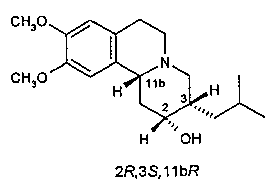 OR 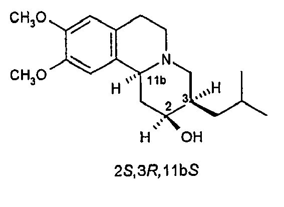 | 3.84 δ 6H (s); | 127.2 δ; | 1611 cm⁻¹; | |
| | 3.15 δ 1H (m); | 112.0 δ; | 1512 cm⁻¹; | |
| | 2.87 δ 3H (m); | 108.3 δ; | 1463 cm⁻¹ | |
| | 2.43 δ 4H (m); | 72.4 δ; | 1362 cm⁻¹; | |
| | 1.81 δ 1H (m); | 61.2 δ; | 1334 cm⁻¹; | |
| | 1.64 δ 4H (m); | 58.3 δ; | 1259 cm⁻¹; | |
| | 1.21 δ 1H (m); | 56.5 δ; | 1227 cm⁻¹; | |
| | 0.94 δ 3H (d); | 56.3 δ; | 1148 cm⁻¹; | |
| | 0.89 δ 3H (d) | 52.7 δ; | 1063 cm⁻¹; | |
| | | 38.6 δ; | 1024 cm⁻¹; | |
| | | 36.7 δ; | 855 cm⁻¹; | |
| | | 34.4 δ; | 766 cm⁻¹ | |
| | | 29.6 δ; | | |
| | | 26.5 δ; | | |
| | | 24.4 δ; | | |
| | | 22.5 δ | | |

Tables 3 and 4 - Chromatography and ORD Data

| Table 3 | | | |
|---|---|---|---|
| Dihydrotetrabenazine isomer | Chiral HPLC Methods and Retention Times | | ORD (MeOH, 21°C) |
| Isomers A and B | Column: | | Isomer A |
| | Chirex (S)-VAL, (R)-NEA, 250 x 4.6 mm | | [α_{D}]-114.6° |
| 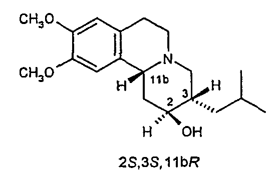 OR 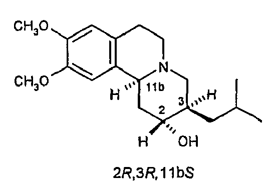 | Mobile phase: ethanol (36:62:2) | Hexane : 1,2-dichloroethane : | |
| | Flow: | 1.0 ml min⁻¹ | Isomer B |
| | UV: | 254 nm | [α_{D}] +123° |
| | Retention times: | | |
| | Isomer A | 16.6 min | |
| | Isomer B | 15.3 min | |

| Table 4 | | | |
|---|---|---|---|
| Isomers C and D | Column: | | Isomer C |
| | Chirex (S)-VAL, (R)-NEA, 250 x 4.6mm | | [α_{D}] +150.9° |
| 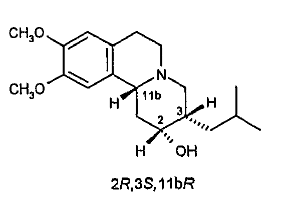 OR 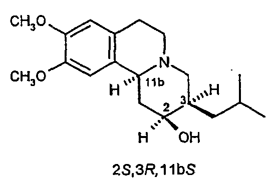 | Mobile phase: | Hexane : ethanol (92:8) | |
| | Flow: | 1.0 ml min⁻¹ | Isomer D |
| | UV: | 254 nm | [α_{D}] -145.7° |
| | Retention times: | | |
| | Isomer C | 20.3 min | |
| | Isomer D | 19.4 min | |

### EXAMPLE 3

### Alternative Method of Preparation of Isomer B and Preparation of Mesylate Salt

### 3A. Reduction of RR/SS Tetrabenazine

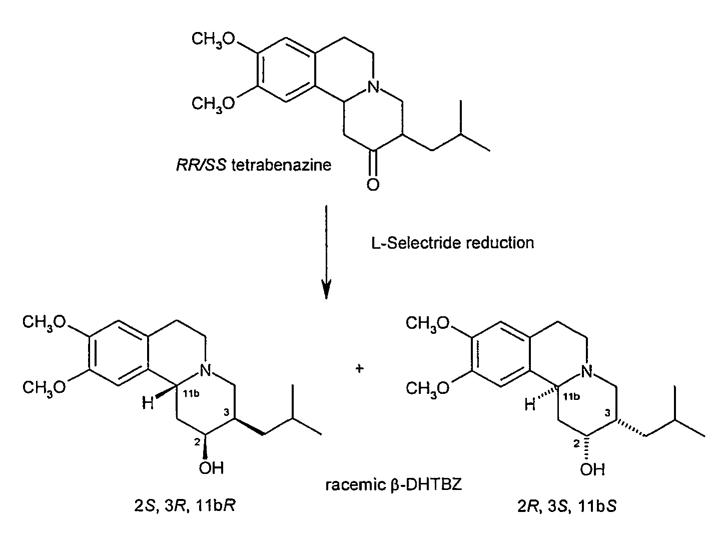

1M L-Selectride^{®} in tetrahydrofuran (52 ml, 52 mmol, 1.1 eq) was added slowly over 30 minutes to a cooled (ice bath), stirred solution of tetrabenazine racemate (15 g, 47 mmol) in tetrahydrofuran (56 ml). After the addition was complete, the mixture was allowed to warm to room temperature and stirred for a further six hours. TLC analysis (silica, ethyl acetate) showed only very minor amounts of starting material remained.

The mixture was poured on to a stirred mixture of crushed ice (112 g), water (56 ml) and glacial acetic acid (12.2 g). The resulting yellow solution was washed with ether (2 x 50 ml) and basified by the slow addition of solid sodium carbonate (ca. 13 g). Pet-ether (30-40 °C) (56 ml) was added to the mixture with stirring and the crude β-DHTBZ was collected as a white solid by filtration.

The crude solid was dissolved in dichloromethane (ca. 150 ml) and the resulting solution washed with water (40 ml), dried using anhydrous magnesium sulphate, filtered and concentrated at reduced pressure to ca. 40 ml. A thick suspension of white solid was formed. Pet-ether (30-40 °C) (56 ml) was added and the suspension was stirred for fifteen minutes at laboratory temperature. The product was collected by filtration and washed on the filter until snow-white using pet-ether (30-40°C) (40 to 60 ml) before air-drying at room temperature to yield β-DHTBZ (10.1 g, 67%) as a white solid. TLC analysis (silica, ethyl acetate) showed only one component.

### 3B. Preparation and Fractional Crystallisation of the Camphorsulphonic acid Salt of Racemic β-DHTBZ

The product of Example 3A and 1 equivalent of (*S*)-(+)-Camphor-10-sulphonic acid were dissolved with heating in the minimum amount of methanol. The resulting solution was allowed to cool and then diluted slowly with ether until formation of the resulting solid precipitation was complete. The resulting white crystalline solid was collected by filtration and washed with ether before drying.

The camphorsulphonic acid salt of (10 g) was dissolved in a mixture of hot absolute ethanol (170 ml) and methanol (30 ml). The resulting solution was stirred and allowed to cool. After two hours the precipitate formed was collected by filtration as a white crystalline solid (2.9 g). A sample of the crystalline material was shaken in a separating funnel with excess saturated aqueous sodium carbonate and dichloromethane. The organic phase was separated, dried over anhydrous magnesium sulphate, filtered and concentrated at reduced pressure. The residue was triturated using pet.-ether (30-40°C) and the organic solution concentrated once more. Chiral HPLC analysis of the salt using a Chirex (S)-VAL and (R)-NEA 250 x 4.6 mm column, and a hexane : ethanol (98:2) eluent at a flow rate of 1 ml/minute showed showed that the isolated β-DHTBZ was enriched in one enantiomer (e.e. ca. 80%).

The enriched camphorsulphonic acid salt (14 g) was dissolved in hot absolute ethanol (140 ml) and propan-2-ol (420 ml) was added. The resulting solution was stirred and a precipitate began to form within one minute. The mixture was allowed to cool to room temperature and stirred for one hour. The precipitate formed was collected by filtration, washed with ether and dried to give a white crystalline solid (12 g).

The crystalline material was shaken in a separating funnel with excess saturated aqueous sodium carbonate and dichloromethane. The organic phase was separated, dried over anhydrous magnesium sulphate, filtered and concentrated at reduced pressure. The residue was triturated using pet.-ether (30-40°C) and the organic solution concentrated once more to yield (after drying in vacuo.) (+)-β-DHTBZ (6.6 g, ORD +107.8°). The isolated enantiomer has e.e. >97%.

### 3C. Preparation of Isomer B

A solution of phosphorus pentachloride (4.5 g, 21.6 mmol, 1.05 eq) in dichloromethane (55 ml) was added steadily over ten minutes to a stirred, cooled (ice-water bath) solution of the product of Example 3B (6.6 g, 20.6 mmol) in dichloromethane (90 ml). When the addition was complete, the resulting yellow solution was stirred for a further ten minutes before pouring on to a rapidly stirred mixture of sodium carbonate (15 g) in water (90 ml) and crushed ice (90 g). The mixture was stirred for a further 10 minutes and transferred to a separating funnel.

Once the phases had separated, the brown dichloromethane layer was removed, dried over anhydrous magnesium sulphate, filtered and concentrated at reduced pressure to give the crude alkene intermediate as brown oil (ca. 6.7 g). TLC analysis (silica, ethyl acetate) showed that no (+)-β-DHTBZ remained in the crude product.

The crude alkene was taken up (dry nitrogen atmosphere) in anhydrous tetrahydrofuran (40 ml) and a solution of borane in THF (1 M solution, 2.5 eq, 52 ml) was added with stirring over fifteen minutes. The reaction mixture was then stirred at room temperature for two hours. TLC analysis (silica, ethyl acetate) showed that no alkene intermediate remained in the reaction mixture.

A solution of sodium hydroxide (3.7 g) in water (10 ml) was added to the stirring reaction mixture, followed by an aqueous solution of hydrogen peroxide (50%, ca. 7 ml) and the two-phase mixture formed was stirred at reflux for one hour. TLC analysis of the organic phase at this time (silica, ethyl acetate) showed the appearance of a product with Rf as expected for Isomer B. A characteristic nonpolar component was also seen.

The reaction mixture was allowed to cool to room temperature and was poured into a separating funnel. The upper organic layer was removed and concentrated under reduced pressure to remove the majority of THF. The residue was taken up in ether (stabilised (BHT), 75 ml), washed with water (40 ml), dried over anhydrous magnesium sulphate, filtered and concentrated under reduced pressure to give a pale yellow oil (8.1 g).

The yellow oil was purified using column chromatography (silica, ethyl acetate : hexane (80:20), increasing to 100% ethyl acetate) and the desired column fractions collected, combined and concentrated at reduced pressure to give a pale oil which was treated with ether (stabilised, 18 ml) and concentrated at reduced pressure to give Isomer B as a pale yellow solid foam (2.2 g).

Chiral HPLC using the conditions set out in Example 3B confirmed that Isomer B had been produced in an enantiomeric excess (e.e.) of greater than 97%.

The optical rotation was measured using a Bellingham Stanley ADP220 polarimeter and gave an [α_{D}] of +123.5°.

### 3D. Preparation of the Mesylate salt of Isomer B

The methanesulphonate salt of Isomer B was prepared by dissolving a mixture of 1 equivalent of Isomer B from Example 3C and 1 equivalent of methane sulphonic acid in the minimum amount of ethanol and then adding diethyl ether. The resulting white precipitate that formed was collected by filtration and dried *in vacuo* to give the mesylate salt in a yield of ca. 85% and a purity (by HPLC) of ca. 96%.

### EXAMPLE 4

### Screen for VMAT-2 binding activity using a [³H] Dihydrotetrabenazine binding assay

Dihydrotetrabenazine is a very potent and selective inhibitor of VMAT-2, and binds with high affinity (nM range) to this vesicular transporter. [³H] Dihydrotetrabenazine has been successfully used for many years as a radioligand to label VMAT-2 in human, bovine and rodent brain (e.g. Scherman et al. J. Neurochem. 50, 1131-1136 (1988); Near et al. Mol. Pharmacol. 30, 252-257 (1986); Kilbourn et al. Eur. J. Pharmacol. 278, 249-252 (1995); and Zucker et al. Life Sci. 69, 2311-2317 (2001)).

The four dihydrotetrabenazine isomers A, B, C and D were tested for their ability to inhibit the VMAT-2 transporter using the assay described below.

### Methods and Materials

Adult rat (Wistar strain) forebrain membranes were prepared essentially as described by Chazot et al. (1993) Biochem. Pharmacol. 45, 605-610. Adult rat striatal vesicular membranes were prepared essentially as described by Roland et al. (2000),JPET 293, 329-335. 10 µg Membranes were incubated at 25°C with [³H] dihydrotetrabenazine (18-20 nM) in 50mM HEPES pH 8.0 (assay buffer), for 60 minutes, and bound radioligand was collected by rapid filtration under vacuum on GF/B glass-fibre filters. Non-specific binding was determined in parallel samples in the presence of 2 µM unlabelled tetrabenazine. Radioactivity was counted in scintillation fluid in a β-counter. A full concentration range (log and half-log units) of four test compounds (Isomers A, B, C and D) were assayed (range: 10⁻¹¹- 10⁻⁴M) in triplicate. Test compounds and tetrabenazine were dissolved in DMSO at a stock concentration of 10 mM, and dilutions then prepared in assay buffer. Three independent experiments were performed for each compound. Data were analysed and curve fitted using the GraphPad Prism 3.2 package.

### Results

Initially, an adult rat forebrain P₂ membrane preparation (Chazot *et al*., 1993) was prepared and was assayed as described in the original protocol. This yielded a very low level of specific binding activity.

An adult rat striatal vesicular preparation was then prepared, which yielded a significant level of stable specific [³H] Dihydrotetrabenazine binding sites (5-6 pmol/mg protein). This compares well with published data (Roland *et al*., 2000) This preparation was utilised for all subsequent assays.

**Table 5 - Competition binding parameters for test compounds**

| Compound | Apparent pIC₅₀ | Overall K_{I} nM) | n_{H} |
|---|---|---|---|
| Isomer A | < 4.0 | < 5,900 | -0.40 ±0.08 |
| | **Two site fit** | | **% sites** |
| | -5.98 ± 0.33 | | 47% |
| | <-4.0 | | 53% |
| Isomer B | -5.63 ± 0.05 | 139 ± 30 | -0.52 ± 0.06 |
| | **Two-site fit** | | **% sites** |
| | -5.15 ± 0.11 | | 74% |
| | -7.13 ± 0.26 | | 26% |
| Isomer C | -6.32 ± 0.02 | 28 ± 2 | -0.77 ± 0.07 |
| Isomer D | -5.13 ± 0.07 | 440 ± 23 | -0.72 ± 0.05 |

Data are mean ± SD for three independent experiments. K_{I} values were determined based on a published rat striatal K_{D} value of 1.2 nM (Roland *et al*., 2000).

The overall pharmacological profile in terms of overall K_{I} values is Isomer C > Isomer B > Isomer D >> Isomer A.

Notably, both Isomer B and Isomer A yielded shallow competition curves, which were best fitted to a two-site binding model.

Isomer A displayed a high affinity (K_{I} = 59 nM) and low affinity site (K_{I} < 5.9 µM affinity), each contributing to approx. 50% of the total sites. This may indicate that Isomer A can differentiate between different striatal VMAT-2 binding sites.

### EXAMPLE 5

### VMAT Functional Assays

### A. VMAT2 Functional Assay

Rat striatal synaptic vesicles were prepared essentially as described in Example 3. Thus, a rat striatal P₂ membrane preparation (Chazot *et al*., 1993) was resuspended and homogenised in ice-cold distilled water. Osmolarity was restored by addition of 25 mM HEPES and 100 mM potassium tartrate (pH 7.5, 4C). The preparation was then centrifuged for 20 minutes at 20,000 x g (4 °C). The resultant S₃ fraction was removed, magnesium sulphate was added (to give a final concentration of 1 mM, pH 7.5, 4°C), and the mixture was centrifuged at 100,000 x g for 45 minutes. The final P₄ fraction contains the synaptic vesicles for the assay.

An aliquot of 100 µl (approx. 2.5 µg protein) of synaptic vesicles was preincubated with increasing concentrations of test compounds C and B (prepared fresh as a stock of 10⁻² M in DMSO) for 30 minutes (concentration range 10⁻⁹ M -10⁻⁴ M), and then for 3 minutes in the assay buffer (25 mM HEPES, 100 mM potassium tartrate, 1.7 mM ascorbic acid, 0.05 mM EGTA, 0.1 mM EDTA, 2 mM ATP-Mg ²⁺, pH 7.5), in the presence of [³H] dopamine (30 nM final concentration) at 30 °C. The reaction was then terminated by addition of ice-cold buffer assay buffer pH 7.5, containing 2 mM MgSO₄ instead of 2 mM ATP-Mg ²⁺, and rapid filtration achieved through Whatman filters soaked in 0.5% polyethyleneimine. The filters were washed three times with cold buffer using a Brandel Harvester. The radioactivity trapped on the filters was counted using a liquid scintillation counter and non-specific binding was determined by measuring vesicular [³H] dopamine uptake at 4 °C. The method was based on that described in Ugarte YV et al. (2003) Eur. J. Pharmacol. 472, 165-171. Selective VMAT-2 uptake was defined using 10 µM tetrabenazine.

Both Compound C (apparent IC₅₀ = 18 ± 2 nM) and Compound B (apparent IC₅₀ = 30 ± 3 nM) inhibited [³H] dopamine uptake into rat striatal vesicles via the VMAT-2 transporter with functional affinities (profile C > B) similar to their respective binding affinities determined using the [³H] Dihydrotetrabenazine binding assay.

### B. VMAT1 functional assay

There are very limited native tissues which possess VMAT1 alone, in isolation from VMAT2. However, tetrabenazine displays at least a 200-fold higher affinity for VMAT2 in comparison to VMAT1, and this discrimination can be used to block the influence of VMAT2 in the functional assay (Erickson et al. (1996) PNAS (USA) 93, 5166-517.1). Adrenal chromaffin cells were isolated from young adult SD rats essentially as described in Moshharov et al. (2003) J Neurosci. 23, 5835-5845. Thus, adrenal glands were dissected in ice cold PBS, the capsule and cortex of the glands removed and the remaining medullae were minced. After multiple washes with PBS, the tissue was incubated with Ca2+-free collagenase IA solution (250U/ml) for 30 minutes at 30 °C with gentle stirring. The digested tissue was rinsed three times and the dissociated cells were centrifuged at 3000 rpm to form a pellet, which was resuspended in PBS. The vesicular fraction was isolated in an identical fashion to that described for the brain preparation.

100 µl (approx. 2.5 µg protein) of synaptic vesicles were preincubated with increasing concentrations of test compound (prepared as previously described for binding assay) for 30 minutes (concentration range 10⁻⁹ M - 10⁻⁴ M). The assay was performed for 3 minutes at 30 °C in the assay buffer (25 mM HEPES, 100 mM potassium tartrate, 1.7 mM ascorbic acid, 0.05 mM EGTA, 0.1 mM EDTA, 2 mM ATP-Mg ²⁺, pH 7.5), in the presence of [³H] dopamine (30 nM final concentration). [³H] dopamine uptake was measured in the presence of 10 µM tetrabenazine (selectively blocks VMAT2 at this concentration). Non-specific uptake was determined by measuring vesicular [³H] dopamine uptake at 4°C. The reaction was then terminated by addition of ice-cold buffer assay buffer pH 7.5, containing 2 mM MgSO₄ instead of 2 mM ATP-Mg ²⁺, and rapid filtration achieved through Whatman filters soaked in 0.5% polyethyleneimine. The filters were washed three times with cold buffer using a Brandel Harvester and the radioactivity trapped on the filters was counted using a liquid scintillation counter.

In the presence of 10 µM Tetrabenazine, both Compound B and Compound C poorly inhibited [³H] dopamine uptake, the IC₅₀ values being greater than 10⁻⁵M for both compounds. This indicates that both compounds have a low affinity for VMAT-1. Moreover, the data show that both compounds have at least 2-orders of magnitude selectivity for VMAT-2 over VMAT-1.

### EXAMPLE 6

### Receptor and Transporter Protein Binding Studies

The four dihydrotetrabenazine isomers A, B, C and D subjected to specific binding assays to test their ability to bind to the receptors and transporter proteins described below. The results are set out in Table 6
**(a) Adrenergic** α**_{2A} Receptor:**
   Reference: S. Uhlçn et al. J. Pharmacol. Exp. Ther., 271:1558-1565 (1994)
   Source: Human recombinant insect Sf9 cells
   Ligand: 1 nM [³H] MK-912
   Vehicle: 1% DMSO
   Incubation time/Temp: 60 minutes @ 25 °C
   Incubation buffer: 75mM Tris-HCl, pH 7.4, 12.5mM MgCl₂, 2mM EDTA
   Non Specific ligand: 10µM WB-4101
   K_{d}: 0.6 nM
   Bₘₐₓ: 4.6 pmole/mg protein
   Specific binding: 95%
   Quantitation method: Radioligand binding
   Significance criteria: ≥ 50% of maximum stimulation or inhibition
**(b) Adrenergic** α**_{2B} Receptor:**
   Reference: S. Uhlen et al., Eur. J. Pharmacol., 33 (1): 93-1-1 (1998)
   Source: Human recombinant CHO-K1 cells
   Ligand: 2.5 nM [3H] Rauwolscine
   Vehicle: 1% DMSO
   Incubation time/Temp: 60 minutes @ 25 °C
   Incubation buffer: 50 mM Tris-HCl, 1 mM EDTA, 12.5mM MgCl₂, pH 7.4, 0.2% BSA at 25 °C
   Non Specific ligand: 10 µM Prazosin
   K_{d}: 2.1 nM
   Bₘₐₓ: 2.1 pmole/mg protein
   Specific binding: 90%
   Quantitation method: Radioligand binding
   Significance criteria: ≥ 50% of maximum stimulation or inhibition
**(c) Dopamine D₁ Receptor:**
   Reference: Dearry et al., Nature, 347:72-76, (1990)
   Source: Human recombinant CHO cells
   Ligand: 1.4 nM [3H] SCH-23390
   Vehicle: 1% DMSO
   Incubation time/Temp: 2 hours @ 37 °C
   Incubation buffer: 50 mM Tris-HCl, pH 7.4, 150 nM NaCl, 1.4 nM ascorbic acid, 0.001% BSA
   Non Specific ligand: 10 µM (+)-butaclamol
   K_{d}: 1.4 nM
   Bₘₐₓ: 0.63 pmole/mg protein
   Specific binding: 90%
   Quantitation method: Radioligand binding
   Significance criteria: ≥ 50% of maximum stimulation or inhibition
**(d) Dopamine D_{2L} Receptor:**
   Reference: Bunzo et al., Nature, 336:783-787 (1988)
   Source: Human recombinant CHO cells
   Ligand: 0.16 nM [3H] Spiperone
   Vehicle: 1% DMSO
   Incubation time/Temp: 2 hours @ 25 °C
   Incubation buffer: 50 mM Tris-HCl, pH 7.4, 150 nM NaCl, 1.4 nM ascorbic acid, 0.001% BSA
   Non Specific ligand: 10 µM Haloperidol
   K_{d}: 0.08 nM
   Bₘₐₓ: 0.48 pmole/mg protein
   Specific binding: 85%
   Quantitation method: Radioligand binding
   Significance criteria: ≥ 50% of maximum stimulation or inhibition
**(e) Dopamine D₃ Receptor:**
   Reference: Sokoloff et al., Nature, 347:146-151, (1990)
   Source: Human recombinant CHO cells
   Ligand: 0.7 nM [3H] Spiperone
   Vehicle: 1% DMSO
   Incubation time/Temp: 2 hours @ 37 °C
   Incubation buffer: 50 mM Tris-HCl, pH 7.4, 150 nM NaCl, 1.4 nM ascorbic acid, 0.001% BSA
   Non Specific ligand: 25 µM S(-)-Sulpiride
   K_{d}: 0.36 nM
   Bₘₐₓ: 1.1 pmole/mg protein
   Specific binding: 85%
   Quantitation method: Radioligand binding
   Significance criteria: ≥ 50% of maximum stimulation or inhibition
**(f) Imidazoline I₂ (Central) Receptor:**
   Reference: Brown et al., Brit. J. Pharmacol., 99:803-809, (1990)
   Source: Wistar rat cerebral cortex
   Ligand: 2 nM [3H] Idazoxan
   Vehicle: 1% DMSO
   Incubation time/Temp: 30 minutes @ 25°C
   Incubation buffer: 50 mM Tris-HCl, 0.5 mM EDTA, pH 7.4 at 25 °C
   Non Specific ligand: 1 µM Idazoxan
   K_{d}: 4 nM
   Bₘₐₓ: 0.14 pmole/mg protein
   Specific binding: 85%
   Quantitation method: Radioligand binding
   Significance criteria: ≥ 50% of maximum stimulation or inhibition
**(g) Sigma** σ**₁ Receptor:**
   Reference: Ganapathy et al., Pharmacol. Exp. Ther., 289:251-260, (1999)
   Source: Human jurkat cells
   Ligand: 8 nM [3H] Haloperidol
   Vehicle: 1% DMSO
   Incubation time/Temp: 4 hours @ 25°C
   Incubation buffer: 5 mM K2HPO4/KH2PO4 buffer pH 7.5
   Non Specific ligand: 10 µM Haloperidol
   K_{d}: 5.8 nM
   Bₘₐₓ: 0.71 pmole/mg protein
   Specific binding: 80%
   Quantitation method: Radioligand binding
   Significance criteria: ≥ 50% of maximum stimulation or inhibition
**(h) Sigma** σ**₂ Receptor:**
   Reference: Hashimoto et al., Eur. J. Pharmacol., 236:159-163, (1993)
   Source: Wistar rat brain
   Ligand: 3 nM [3H] Ifenprodil
   Vehicle: 1% DMSO
   Incubation time/Temp: 60 minutes @ 37 °C
   Incubation buffer: 50 mM Tris-HCl, pH 7.4
   Non Specific ligand: 10 µM Ifenprodil
   K_{d}: 4.8 nM
   Bₘₐₓ: 1.3 pmole/mg protein
   Specific binding: 85%
   Quantitation method: Radioligand binding
   Significance criteria: ≥ 50% of maximum stimulation or inhibition
**(i) Serotonin Transporter (SERT):**
   Reference: Gu et al., J. Biol. Chem., 269(10):7124-7130, (1994)
   Source: Human recombinant HEK-293 cells
   Ligand: 0.15 nM [125I] RTI-55
   Vehicle: 1% DMSO
   Incubation time/Temp: 3 hours @ 4 °C
   Incubation buffer: 100 mM NaCl, 50 mM Tris HCl, 1 µM Leupeptin, 10 µM PMSF, pH 7.4
   Non Specific ligand: 10 µM Imipramine
   K_{d}: 0.17 nM
   Bₘₐₓ: 0.41 pmole/mg protein
   Specific binding: 95%
   Quantitation method: Radioligand binding
   Significance criteria: ≥ 50% of maximum stimulation or inhibition
**(j) Dopamine Transporter (DAT):**
   Reference: Giros et al., Trends Pharmacol. Sci., 14, 43-49 (1993)
      Gu et al., J. Biol. Chem., 269(10):7124-7130 (1994)
   Source: Human recombinant CHO cells
   Ligand: 0.15 nM [¹²⁵I] RTI-55
   Vehicle: 1% DMSO
   Incubation time/Temp: 3 hours @ 4 °C
   Incubation buffer: 100 mM NaCl, 50 mM Tris HCl, 1 µM Leupeptin, 10 µM PMSF, pH 7.4
   Non Specific ligand: 10 µM Nomifensine
   K_{d}: 0.58 nM
   Bₘₐₓ: 0.047 pmole/mg protein
   Specific binding: 90%
   Quantitation method: Radioligand binding
   Significance criteria: ≥ 50% of maximum stimulation or inhibition

**Table 6**

| Percentage Inhibition by 10 µM Solutions of Dihydrotetrabenazine isomers of Specific Binding at Receptor and Transporter Proteins (IC₅₀ value, where measured, is in parentheses) | | | | |
|---|---|---|---|---|
| Receptor/Protein | Isomer A | Isomer B | Isomer C | Isomer D |
| (a) α_{2A} Receptor | 86 | 12 | 13 | 87 |
| (b) α_{2B} Receptor | 44 | 14 | -7 | 50 |
| (c) D₁ Receptor | 78 | 1 | 6 | 38 |
| (d) D_{2L} Receptor | 87 | 16 | -14 | 58 |
| (e) D₃ Receptor | 69 | 7 | 9 | 63 |
| (f) I₂ Receptor | 74 | 8 | 0 | 55 |
| (g) σ₁ Receptor | 48 | 82 | 59 | 82 |
| (h) σ₂ Receptor | 64 | 64 | 61 | 69 |
| (i) SERT | 19 | 86 (0.35) | 77 (2.75) | 8 |
| (j) DAT | 3 | 4 | -2 | 2 |

### EXAMPLE 7

### Enzyme Assays

Isomers B and C were tested for their ability to inhibit enzymes involved in the processing of monoamines in the CNS, namely Catechol O-Methyl Transferase (COMT), Monoamine Oxidase A and Monoamine Oxidase B. The assay methods used are described below and the results are set out in Table 7.
**(a) Catechol O-Methyl Transferase (COMT) Inhibition**
   Source: Porcine liver
   Substrate: 3 mM catechol + S-adenosyl-L-[³H]methionine
   Vehicle: 1% DMSO
   Pre-incubation time/Temp: None
   Incubation time: 60 minutes @ 37 °C
   Incubation buffer: 100 mM potassium phosphate, 10mM MgCl₂, 3 mM DTT containing 12 units/ml adenosine deaminase, pH 7.4
   Quantitation method: Quantitation of [³H] guiacol.
   Significance criteria: ≥ 50% of maximum stimulation or inhibition
**(b) Monoamine Oxidase MAO-A Inhibition**
   Source: Human recombinant
   Substrate: 50 µM kynuramine
   Vehicle: 1% DMSO
   Pre-incubation time/Temp: 15 minutes @ 37 °C
   Incubation time: 60 minutes @ 37 °C
   Incubation buffer: 100 mM KH₂PO₄, pH 7.4
   Quantitation method: Spectrofluorimetric quantitation of 4-hydroxyquinoline
   Significance criteria: ≥ 50% of maximum stimulation or inhibition
**(c) Monoamine Oxidase MAO-B Inhibition**
   Source: Human recombinant
   Substrate: 50 µM kynuramine
   Vehicle: 1% DMSO
   Pre-incubation time/Temp: 15 minutes @ 37 °C
   Incubation time: 60 minutes @ 37 °C
   Incubation buffer: 100 mM KH₂PO₄, pH 7.4
   Quantitation method: Spectrofluorimetric quantitation of 4-hydroxyquinoline
   Significance criteria: ≥ 50% of maximum stimulation or inhibition

**Table 7**

| Percentage Inhibition of Enzyme Activity by 10 µM Solutions of Dihydrotetrabenazine isomers | | | | |
|---|---|---|---|---|
| Enzyme | Isomer A | Isomer B | Isomer C | Isomer D |
| (a) COMT | | -12 | -22 | |
| (b) MAO-A | | 3 | 3 | |
| (c) MAO-B | | -5 | -5 | |

### EXAMPLE 8

### Cellular Assays

The ability of isomers B and C to inhibit uptake of serotonin (5-hydroxytryptamine) by human embryonic kidney cells was measured using the following assay conditions:
Target: Human HEK-293 cells
Vehicle: 0.4 % DMSO
Incubation Time/Temp: 10 minutes @ 25 °C
Incubation buffer: 5mM Tris-HCl, 7.5 mM HEPES, 120 mM NaCl, 5.4 mM KCl, 1.2 mM CaCl₂, 1.2 mM MgSO₄, 5 mM glucose, 1 mM ascorbic acid, pH 7.1
Quantitation Method: Quantitation of [³H] serotonin uptake
Significance criteria: ≥ 50% inhibition of [³H] serotonin uptake relative to fluxetine response.

### Results

Compound B was shown to be an antagonist and produced 56 % inhibition of serotonin uptake at a concentration of 10 µM. Compound B had an IC₅₀ of 7.53 µM.

Compound C was also shown to be an antagonist and produced 86 % inhibition of serotonin uptake at a concentration of 10 µM. Compound B had an IC₅₀ of 1.29 µM.

### EXAMPLE 9

### 5-HT _{1D/1B} Binding Assay

The ability of Compound B and Compound C to bind to 5-HT _{1D/1B} receptors was tested using an assay based on the one described by Millan, MJ et al. (2002) Pharmacol. Biochem. Behav. 71, 589-598. [N-methyl ³H] GR-125743 was used as the radioliogand for both 5-HT_{1D} and 5-HT_{1B} receptors. Adult SD rat forebrain P₂ membranes (Chazot *et al*., 1993) were used for the assay. The assay buffer used was 50 mM Tris-HCl pH 7.7 at room temperature containing 4 mM calcium chloride, 0.1% ascorbic acid and 10 µM pargyline. 5-HT (10 µM) was used to define non-specific binding. Incubation with 1 nM [³H] GR-125743 was carried out for 1 hour at room temperature, and the reaction was terminated by rapid filtration using a Brandel Harvester through GF/B filters pre-soaked in 0.1% polyethyleneimine, followed by three washes with ice-cold buffer (supplemented with 0.1% BSA). A dose range of 10⁻¹⁰-10⁻⁴M was utilised. The resultant competition curves were analysed using the GraphPad Prism 4 package.

Both compounds B and C displayed poor displacement of [³H] [N-methyl] GR-125743 binding to rat forebrain membranes (IC₅₀ values > 10⁻⁴M), suggesting that both B and C have a low affinity for 5-HT _{1D/B} receptors.

### EXAMPLE 10

### Determination Of The Intestinal Permeability of Dihydrotetrabenazine Isomers A, B, C and D using The Caco-2 Absorption Assay

The Caco-2 absorption assay is a well-established system for the *in vitro* estimation of *in vivo* intestinal absorption of drugs - see Meunier et al., Cell Biology and Toxicology, 11:187-194, Wils et al., Cell Biology and Toxicology, 10:393-397, and Gres et al., Pharm Sci, 15(5):726-732.

The assay relies on the ability of Caco-2 cells to differentiate into enterocytes when cultured on a microporous filter for a period of approximately 21 days. During the culture period, the Caco-2 cells undergo spontaneous morphological and biochemical changes, which produce a polarized monolayer with a well-defined brush border on the apical surface, as well as tight cellular junctions. Therefore, these cells may be used as an *in vitro* model for the analysis of drug permeability.

Absorption across the Caco-2 monolayer can be measured in two directions: apical-to-basolateral or basolateral-to-apical, by adding the compound to the apical or the basolateral chamber, respectively. At various time-points, samples are collected from the receiver-chamber for analysis for the rate of absorption as measured by the apparent permeability coefficient (*Papp*) across the monolayer.

The *Papp* value reflects a combination of the test article permeability through both transcellular (through cell membranes) and the paracellular (across the tight junctions between the cells) pathways. The relative contribution of these pathways depends upon the pKa, partition coefficient (log D), molecular radius, and charge of the test article at a given pH. *Papp* values can then be used to rank-order compounds for their permeability through Caco-2 monolayers.

The apparent permeability coefficients (*Papp*) of the four dihydrotetrabenazine isomers A, B, C and D at 50 µM were determined using the Caco-2 absorption model and ranked in relation to reference compounds of low, medium and high permeability. The radio-labelled reference standards mannitol (low), salicylic acid (medium) and testosterone (high) were used to establish a rank order of permeability. The *Papp* value of each dihydrotetrabenazine test article was estimated by measuring its concentration in the donor and receiver compartments after 1 hour. Absorption was determined at pH 7.4 across cell monolayers in the apical-to-basolateral direction. Caco-2 cells used to estimate *Papp* values were grown for 26 days on Transwell inserts in 12-well plates. Monolayer integrity was verified before and after the absorption assay by the methods of transepithelial electrical resistance (Ohm-cm²) and Lucifer Yellow.

### Materials and Methods

Stock solutions of mannitol, salicylic acid and testosterone were prepared in methanol at 50 mM. On the day of the assay, the reference standards were diluted in Hank's balanced salt solution (HBSS), pH 7.4 to a final concentration of 50 µM. Radio-labelled ¹⁴C-mannitol, ³H-testosterone and ¹⁴C-salicylic acid were then added to their corresponding un-labelled media to have a final specific activity of 0.35 - 0.65 µCi/mL. Dihydrotetrabenazine isomer samples were prepared at a concentration of 50 mM in DMSO. Each stock solution was further diluted in HBSS buffer (pH 7.4) to a final working concentration of 50 µM

The Caco-2 cell suspensions were prepared as follows. One vial of Caco-2 initial cell ID No. Caco-29-080299 from passage number 29 (American Type Culture Collection (VA, USA) was thawed and put in culture in a 150 cm² flask containing Caco-2 culture medium. At confluency, the culture medium was removed and cells were washed with 5 ml of PBS. Cells were detached following the addition and incubation with 0.25 % trypsin-EDTA (2.0 ml per flask) for approximately 10 minutes at 37 °C. Detachment of cells was monitored under a microscope and stopped by the addition of 10 mL of Caco-2 culture medium. Cell viability and concentration were assessed by the trypan blue exclusion method. Once the cell density and viability were determined, Caco-2 cells were diluted in culture medium to a final working concentration of 2.0 x 10⁵ cells/ml. The Caco-2 culture medium contained Dulbecco's Modified Eagle Medium, 10% fetal bovine serum, 100 µM non-essential amino acids, 100 U/ml penicillin and 100 µg/ml streptomycin.

Costar polycarbonate membranes (0.4 µm pore size) were pre-equilibrated with Caco-2 culture medium for 1 hour in a 37 °C water-jacketed incubator with 5% CO₂. The content of the apical chamber was removed and replaced with 500 µl of Caco-2 cell suspension (200 000 cells/ml). Cells were further maintained in culture for 26 days in a 37 °C water jacketed incubator with 5% CO₂.

Prior to the assay, all monolayers were washed twice with HBSS and their transepithelial electrical resistance (TEER) was measured with a Millicell-ERS meter. TEER values obtained in the absence of cells were subtracted as background signal. Only monolayer with TEER values over 150 Ohm-cm² were used for the absorption assay experiment.

All absorption experiments were conducted in triplicate at pH 7.4. The rate of absorption of each reference standard and dihydrotetrabenazine test article was assessed in the apical-to-basolateral direction. Aliquots (100 µl) of each working solution (reference standards and test articles at 50 µM) were set aside at the beginning of the assay for the determination of the initial concentration (Cₒ).

Absorption experiments were initiated by replacing the content of the donor chamber with 500 µl of Hanks Balanced Salt Solution containing test articles or reference compounds. Cells were returned to the CO₂ incubator for the absorption assay. Samples of 100 µl were collected from the receiver and donor chambers after 1 hour and were used to determine the percentage of recovery. Radio-labelled reference standards, mannitol, salicylic acid and testosterone were used as quality controls and for the comparative ranking of dihydrotetrabenazine test articles.

At the end of the absorption experiment, the integrity of each cell monolayer was assessed by monitoring the leakage of Lucifer Yellow from the apical-to-basolateral side. All solutions were removed from the apical and basolateral chambers. The basolateral chambers were replenished with 1.5 ml of fresh HBSS, while the apical chambers were filled with 0.5 ml of 120 µg/ml Lucifer Yellow solution. Cells were returned to the incubator for a period of 1 hour, after which samples of 100 µl were collected and quantified spectrophotometrically with the SpectraMax 340PC-plate reader at 428 nm.

A volume of 20 µl of each radio-labelled sample was added to 10 ml of scintillation cocktail (ScintiSafe 30%) and counted for up to 5 minutes with a liquid scintillation analyzer (1900CA Tri-Carb).

Caco-2 cell incubations were analyzed for the presence of dihydrotetrabenazine using a liquid chromatography-tandem/mass spectrometry (LC-MS/MS) method.

The apparent permeability coefficients were calculated using the equation *Papp* = dQ/dt x 1/A x 1/Cₒ (cm/sec) in which dQ/dt is the rate of diffusion of compound (µg/sec or integration area/sec), A is the total cell membrane surface area (cm²) and Cₒ is the initial concentration (µg/mL or integration area/sec).

The apical-to-basolateral *Papp* values of the different dihydrotetrabenazine test articles were compared with the *Papp* values determined for the reference standards. The results are shown in table 8. Compounds C, D and A of dihydrotetrabenazine have a respective *Papp* value of 21.14 x 10⁻⁶, 24.87 x 10⁻⁶ and 25.52 x 10⁻⁶ cm/sec, which is comparable to the testosterone reference standard.

Compound B has a *Papp* value of 11.98 x 10⁻⁶ cm/sec, which is similar to the salicylic acid reference standard.

**Table 8**

| **Compound Name** | **Direction** | **TEER (Ohm-cm²)** | **Lucifer Yellow (%/hr)** | **Recovery (%)** | **1-hr *Papp* (cm/sec) × 10⁻⁶** |
|---|---|---|---|---|---|
| Mannitol | A-to-B | 379 ± 8 | BLQ | 97.1 ± 0.3 | 1.18 ± 0.14 |
| Salicylic Acid | A-to-B | 407 ± 22 | 1.02 | 91.5 ± 11.4 | 8.91 ± 8.14 |
| Testosterone | A-to-B | 401 ± 26 | 0.28 | 105.0 ± 10.6 | 29.10 ± 7.05 |
| Compound B | A-to-B | 367 ± 15 | BLQ | 90.1 ± 6.5 | 11.98 ± 5.26 |
| Compound C | A-to-B | 390 ± 14 | BLQ | 101.5 ± 12.4 | 21.14 ± 8.65 |
| Compound D | A-to-B | 414 ± 54 | BLQ | 106.5 ± 23.8 | 24.87 ± 14.76 |
| Compound A | A-to-B | 409 ± 68 | BLQ | 106.8 ± 7.9 | 25.52 ± 5.92 |

Compounds C, D and A have a *Papp* value close to the testosterone value, indicating that these compounds have high permeability in the caco-2 model whereas Compound B has a *Papp* value between the salicylic acid and testosterone values, indicating that it has a medium permeability in the Caco-2 model. These results suggest that the four dihydrotetrabenazine isomers should be highly absorbed through the intestinal epithelium *in vivo*.

### EXAMPLE 11

### Comparison of the Sedative Properties of Tetrabenazine and the Dihydrotetrabenazine Isomers B and C

A study was carried out in rats to determine whether the dihydrotetrabenazine isomers of the invention have sedative properties. The effects of the isomers on spontaneous locomotor activity in rats were compared with the effects produced by tetrabenazine and haloperidol using the methods set out below.

### Methods

Male Sprague-Dawley rats, (Charles River Laboratories, Saint-Germain/L'Arbresle, France), weighing 200-250 g at the beginning of the study, were used for the studies. The rats were housed, 2 or 3 per cage, in Makrolon type III cages, in a room set up with the following environmental conditions: temperature: 20 ± 2 °C, humidity : minimum 45 %, air changes: > 12 per hour, light/dark cycle of 12 h/12 h [on at 7:00 a.m.]. The rats were allowed to acclimatize to their conditions for at least five days before commencement of the study. The rats received food (Dietex, Vigny, France, ref. 811002) and water (tap water in water bottle) *ad libitum*.

Solutions of each test compound in corn oil were freshly prepared on the day of the experiment. Haloperidol was prepared in hydroxyethylcellulose, 0.5% in deionized water. Either the vehicle or the test compounds were administered as a single dose (0.3, 1, 3 and 10 mg/kg, 2 mL/kg i.p.). The reference compound haloperidol (1 mg/kg) was administered i.p. (2 mL/kg).

The animals were placed in plexiglass cages under a video camera in a room with low light intensity (maximum 50 lux). At forty five minutes and 3 hours after administration, locomotor activity was determined during 20 minute periods using a video image analyzer (Videotrack, View Point, France). Locomotor activity was recorded in the reference group (haloperidol) at 1 hour after administration. The number and duration of ambulatory movements and duration of inactivity was measured. At the end of the locomotor activity measurement (45 minutes and 3 hours), palpebral closure and arousal were be scored as follows in the plexiglass cage :
Palpebral closure :
   0 : (normal) eyelids wide open
   1 : eyelids slightly drooping
   2 : ptosis, drooping eyelids approximately half-way
   3 : eyelids completely shut
Arousal :
   1 : very low, stupor, coma, little or no responsiveness
   2 : low, some stupor, « dulled », some head or body movement
   3 : somewhat low, slight stupor, some exploratory movements with periods of immobility
   4 : normal, alert, exploratory movements /slow freeze
   5 : somewhat high, slight excitement, tense, sudden darting or freezing
   6 : very high, hyper alert, excited, sudden bouts of running or body movements

The number of occurrences and duration (in seconds) of ambulatory (large) movements and the duration of periods of inactivity (seconds) was determined during two 20 minute periods (45 minutes and 3 hours after administration) using a video image analyzer (Videotrack, ViewPoint, Lyon, France). Image tracking was performed using a video camera placed above the plexiglass cage, recording overall locomotor activity. Images recorded with the video camera were digitalized and displacement of the centre of gravity of the digital image spots was tracked and analyzed using the following method: the speed of displacement of the centre of gravity of the spot was measured and two threshold values were set to define the type of movement: threshold 1 (high speed) and threshold 2 (low speed). When the animal moved and the speed of displacement of the centre of gravity of the spot was above threshold 1, the movement was considered as an ambulatory movement. When the animal remained inactive, the speed was below threshold 2, the movement was considered as inactivity.

The results were expressed as the means ± SEMs of the 12 individual values. Statistical analyses were carried out using ANOVA (one way) and Dunnett's t-test and with the non parametric test of Kruskal-Wallis followed by a Mann & Whitney U-test for the sedation cotation. A p value of p<0.05 was taken as indicating significance..

### Protocol

Group size n=12
Group 1: Reference, haloperidol (1 mg/kg i.p.)
Group 2: Vehicle control group (2 ml/kg i.p.)
Group 3: tetrabenazine (0.3 mg/kg i.p)
Group 4: tetrabenazine (1 mg/kg i.p)
Group 5: tetrabenazine (3 mg/kg i.p)
Group 6: tetrabenazine (10 mg/kg i.p)
Group 7: Isomer C (0.3 mg/kg i.p)
Group 8: Isomer C (1 mg/kg i.p)
Group 9: Isomer C (3 mg/kg i.p)
Group 10: Isomer C (10 mg/kg i.p)
Group 11: Isomer B (0.3 mg/kg i.p)
Group 12: Isomer B (1 mg/kg i.p)
Group 13: Isomer B (3 mg/kg i.p)
Group 14: Isomer B (10 mg/kg i.p)

### Results

| Effects of Tetrabenazine, Isomer B, Isomer C ( 0.3, 1, 3 and 10 mg/kg i.p.) on spontaneous locomotor activity in rats | | | | |
|---|---|---|---|---|
| Observation time : 45 minutes after administration | | | | |
| | | **Large movements** | | **Inactivity** |
| **Treatment** | **Dose (mg/kg)** | **Occurrence** | **Duration (sec)** | **Duration (sec)** |
| Vehicle | 2 mL/kg | 286 ± 35 | 76.4 ± 10.9 | 349.0 ± 37.4 |
| Haloperidol | 1 mg/kg | 58 ± 33 ** | 14.8 ± 8.5 ** | 637.2 ± 60.1 ** |
| Tetrabenazine | 0.3 mg/kg | 253 ± 32 | 66.8 ± 10.7 | 390.4 ± 37.4 |
| Tetrabenazine | 1 mg/kg | 189 ± 32 | 46.5 ± 8.6 | 456.5 ± 50.5 |
| Tetrabenazine | 3 mg/kg | 38 ± 25 ** | 8.7 ± 5.9 ** | 697.8 ± 39.7 ** |
| Tetrabenazine | 10 mg/kg | 1 ± 1 ** | 0.2 ± 0.2 ** | 723.1 ± 46.5 ** |
| Isomer C | 0.3 mg/kg | 285 ± 34 | 79.2 ± 10.0 | 323.7 ± 25.6 |
| Isomer C | 1 mg/kg | 295 ± 30 | 71.8 ± 8.3 | 324.6 ± 38.1 |
| Isomer C | 3 mg/kg | 308 ± 36 | 84.0 ± 9.4 | 322.7 ± 27.8 |
| Isomer C | 10 mg/kg | 254 ± 32 | 66.5 ± 9.9 | 368.7 ± 30.9 |
| Isomer B | 0.3 mg/kg | 268 ± 36 | 72.0 ± 9.6 | 346.1 ± 36.9 |
| Isomer B | 1 mg/kg | 297 ± 22 | 87.0 ± 7.6 | 334.0 ± 23.2 |
| Isomer B | 3 mg/kg | 313 ± 38 | 89.1 ± 12.4 | 342.2 ± 33.3 |
| Isomer B | 10 mg/kg | 298 ± 37 | 84.0 ± 11.2 | 333.1 ± 26.9 |

| Observation time : 3 hours after administration | | | | |
|---|---|---|---|---|
| | | **Large movements** | | **Inactivity** |
| Treatment | Dose (mg/kg) | Occurrence | Duration (sec) | Duration (sec) |
| Vehicle | 2 mL/kg | 101 ± 23 | 24.8 ± 6.0 | 540.9 ± 37.5 |
| Haloperidol | 1 mg/kg | 9 ± 8 ** | 2.2 ± 2.0 ** | 723.6 ± 50.2 ** |
| Tetrabenazine | 0.3 mg/kg | 96 ± 14 | 24.3 ± 4.2 | 545.9 ± 37.1 |
| Tetrabenazine | 1 mg/kg | 90 ± 16 | 21.5 ± 4.0 | 556.9 ± 31.1 |
| Tetrabenazine | 3 mg/kg | 9 ± 4 ** | 1.7 ± 0.9 ** | 729.9 ± 26.8 ** |
| Tetrabenazine | 10 mg/kg | 3 ± 1 ** | 0.6 ± 0.3 ** | 762.1 ± 40.7 ** |
| Isomer C | 0.3 mg/kg | 113 ± 19 | 31.4 ± 6.0 | 519.3 ± 33.7 |
| Isomer C | 1 mg/kg | 128 ± 24 | 30.3 ± 6.5 | 510.2 ± 44.9 |
| Isomer C | 3 mg/kg | 125 ± 22 | 30.2 ± 5.5 | 493.6 ± 38.5 |
| Isomer C | 10 mg/kg | 164 ± 30 | 42.7 ± 8.0 | 465.7 ± 49.0 |
| Isomer B | 0.3 mg/kg | 101 ± 29 | 28.9 ± 9.2 | 566.4 ± 44.3 |
| Isomer B | 1 mg/kg | 125 ± 18 | 34.5 ± 6.2 | 525.8 ± 28.6 |
| Isomer B | 3 mg/kg | 113 ± 17 | 31.1 ± 6.5 | 530.5 ± 38.0 |
| Isomer B | 10 mg/kg | 120 ± 26 | 30.9 ± 6.4 | 515.0 ± 53.0 |

| | | | | |
|---|---|---|---|---|
| **Significantly different from Vehicle group (p,0.01)ANOVA one way followed by Dunnett's test. | | | | |

The results demonstrate that tetrabenazine produces a dose-dependent sedative effect 45 minutes and 3 hours after administration whereas Isomer B and Isomer C show no sedative effects at any time, although isomer C does show a slight and non-significant hyperlocomotor effect 3 hours after administration..

### EXAMPLE 12

### Pharmaceutical Compositions

### (i) Tablet Formulation -I

A tablet composition containing a dihydrotetrabenazine of the invention is prepared by mixing 50mg of the dihydrotetrabenazine with 197mg of lactose (BP) as diluent, and 3mg magnesium stearate as a lubricant and compressing to form a tablet in known manner.

### (ii) Tablet Formulation - II

A tablet composition containing a dihydrotetrabenazine of the invention is prepared by mixing the compound (25 mg) with iron oxide, lactose, magnesium stearate, starch maize white and talc, and compressing to form a tablet in known manner.

### (iii) Capsule Formulation

A capsule formulation is prepared by mixing 100mg of a dihydrotetrabenazine of the invention with 100mg lactose and filling the resulting mixture into standard opaque hard gelatin capsules.

## Claims

1. 3,11b-*cis*-Dihydrotetrabenazine.

2. 3,11b-*cis*-Dihydrotetrabenazine according to claim 1 which is in a (+)-isomeric form.

3. A composition comprising 3,11b-*cis*-dihydrotetrabenazine according to claim 1, the composition being substantially free of 3,11b-*trans*-dihydrotetrabenazine.

4. A composition according to claim 3 wherein the 3,11b-*cis*-dihydrotetrabenazine is a (+)-isomer.

5. 3,11b-*cis*-Dihydrotetrabenazine according to claim 1 which is the 2*S*,3*S*,11b*R* isomer having the formula (Ia):

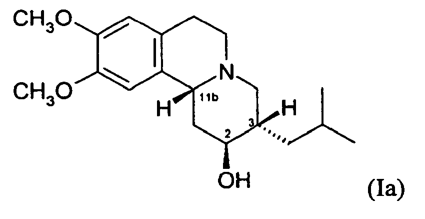

6. 3,11b-*cis*-Dihydrotetrabenazine according to claim 1 which is the 2*R*,3*R*,11b*S* isomer having the formula (Ib):

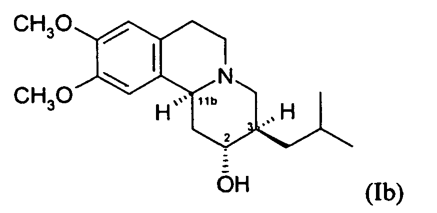

7. 3,11b-*cis*-Dihydrotetrabenazine according to claim 1 which is the 2*R*,3*S*,11b*R* isomer having the formula (Ic):

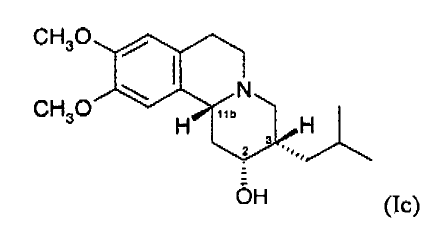

8. 3,1b-*cis*-Dihydrotetrabenazine according to claim 1 which is the 2*S*,3*R*, 11b*S* isomer having the formula (Id):

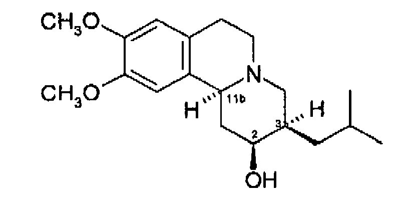

9. 3,11b-*cis*-Dihydrotetrabenazine according to claim 1 in the form of Isomer A which has:
(a) spectroscopic characteristics as follows:
| ¹H-NMR spectrum (CDCl₃) | ¹³C-NMR Mass spectrum (CDCl₃) | IR Spectrum (KBr solid) | Spectrum (ES⁺) |
|---|---|---|---|
| 6.67δ 1H (s); | 147.7δ; | 2950 cm⁻¹; | MH⁺ 320 |
| 6.57 δ 1H (s); | 147.6δ; | 2928 cm⁻¹; | |
| 3.84 δ 6H (s); | 130.5δ; | 2868 cm⁻¹; | |
| 3.55 δ 1H (br. d); | 127.6δ; | 2834 cm⁻¹; | |
| 3.08 δ 1H (m); | 112.1δ; | 1610 cm⁻¹; | |
| 2.79 δ 2H (m); | 108.4δ; | 1511 cm⁻¹; | |
| 2.55 δ 3H (m); | 70.5δ; | 1464 cm⁻¹ | |
| 2.17 δ 1H (m); | 57.5 δ; | 1364 cm⁻¹; | |
| 1.72 δ 6H (m); | 56.5 δ; | 1324 cm⁻¹; | |
| 1.02 δ 1H (m); | 56.3 δ; | 1258 cm⁻¹; | |
| 0.88 δ 6H (t) | 54.8 δ; | 1223 cm⁻¹; | |
| | 53.2 δ; | 1208 cm⁻¹; | |
| | 40.4 δ; | 1144 cm⁻¹; | |
| | 40.1 δ; | 1045 cm⁻¹; | |
| | 36.0 δ; | 1006 cm⁻¹; | |
| | 28.8 δ; | 870 cm⁻¹; | |
| | 26.2 δ; | 785 cm⁻¹; | |
| | 23.7δ; | 764 cm⁻¹ | |
| | 22.9 δ | | |
(b) chromatographic characteristics as follows:
| Chiral HPLC Methods and Retention Times | |
|---|---|
| Column: | |
| Chirex (S)-VAL, (R)-NEA, 250 x 4.6 mm | |
| Mobile phase: (36:62:2) | Hexane : 1,2-dichloroethane : ethanol |
| Flow: | 1.0 ml min⁻¹ |
| UV: | 254 nm |
| Retention time: | |
| Isomer A | 16.6 min |
and
(c) laevorotatory optical activity.

10. 3,11b-*cis*-Dihydrotetrabenazine according to claim 1 in the form of Isomer B which has:
(a) spectroscopic characteristics as follows:
| ¹H-NMR spectrum (CDCl₃) | ¹³C-NMR spectrum (CDCl₃) | IR Spectrum (KBr solid) | Mass Spectrum (ES⁺) |
|---|---|---|---|
| 6.67δ 1H (s); | 147.7δ; | 2950 cm⁻¹; | MH⁺ 320 |
| 6.57 δ 1H (s); | 147.6δ; | 2928 cm⁻¹; | |
| 3.84 δ 6H (s); | 130.5δ; | 2868 cm⁻¹; | |
| 3.55 δ 1H (br. d); | 127.6δ; | 2834 cm⁻¹; | |
| 3.08 δ 1H (m); | 112.1δ; | 1610 cm⁻¹; | |
| 2.79 δ 2H (m); | 108.4δ; | 1511 cm⁻¹; | |
| 2.55 δ 3H (m); | 70.5δ; | 1464 cm⁻¹ | |
| 2.17 δ 1H (m); | 57.5 δ; | 1364 cm⁻¹; | |
| 1.72 δ 6H (m); | 56.5 δ; | 1324 cm⁻¹; | |
| 1.02 δ 1H (m); | 56.3 δ; | 1258 cm⁻¹; | |
| 0.88 δ 6H (t) | 54.8 δ; | 1223 cm⁻¹; | |
| | 53.2 δ; | 1208 cm⁻¹; | |
| | 40.4 δ; | 1144 cm⁻¹; | |
| | 40.1 δ; | 1045 cm⁻¹; | |
| | 36.0 δ; | 1006 cm⁻¹; | |
| | 28.8 δ; | 870 cm⁻¹; | |
| | 26.2 δ; | 785 cm⁻¹; | |
| | 23.7δ; | 764 cm⁻¹ | |
| | 22.9 δ | | |
(b) chromatographic characteristics as follows:
| Chiral HPLC Methods and Retention Times | |
|---|---|
| Column: | |
| Chirex (S)-VAL, (R)-NEA, 250 x 4.6 mm | |
| Mobile phase: (36:62:2) | Hexane : 1,2-dichloroethane : ethanol |
| Flow: | 1.0 ml min⁻¹ |
| UV: | 254 nm |
| Retention time: | |
| Isomer B | 15.3 min |
and
(c) dextrorotatory optical activity.

11. 3,11b-*cis*-Dihydrotetrabenazine according to claim 1 in the form of Isomer C which has:
(a) spectroscopic characteristics as follows:
| ¹H-NMR spectrum (CDCl₃) | ¹³C-NMR spectrum (CDCl₃) | IR Spectrum (KBr solid) | Mass Spectrum (ES⁺) |
|---|---|---|---|
| 6.68 δ 1H (s); | 147.8 δ; | 3370 cm⁻¹; | MH⁺ 320 |
| 6.58 δ 1H (s); | 147.7 δ; | 2950 cm⁻¹; | |
| 3.92 δ 1H (m); | 130.4 δ; | 2929 cm⁻¹; | |
| 3.84 δ 6H (s); | 127.2 δ; | 1611 cm⁻¹; | |
| 3.15 δ 1H (m); | 112.0 δ; | 1512 cm⁻¹; | |
| 2.87 δ 3H (m); | 108.3 δ; | 1463 cm⁻¹ | |
| 2.43 δ 4H (m); | 72.4 δ; | 1362 cm⁻¹; | |
| 1.81 δ 1H (m); | 61.2 δ; | 1334 cm⁻¹; | |
| 1.64 δ 4H (m); | 58.3 δ; | 1259 cm⁻¹; | |
| 1.21 δ 1H (m); | 56.5 δ; | 1227 cm⁻¹; | |
| 0.94 δ 3H (d); | 56.3 δ; | 1148 cm⁻¹; | |
| 0.89 δ 3H (d) | 52.7 δ; | 1063 cm⁻¹; | |
| | 38.6 δ; | 1024 cm⁻¹; | |
| | 36.7 δ; | 855 cm⁻¹; | |
| | 34.4 δ; | 766 cm⁻¹ | |
| | 29.6 δ; | | |
| | 26.5 δ; | | |
| | 24.4 δ; | | |
| | 22.5 δ | | |
(b) chromatographic characteristics as follows:
| Column: | |
|---|---|
| Chirex (S)-VAL, (R)-NEA, 250 x 4.6mm | |
| Mobile phase: | Hexane : ethanol (92:8) |
| Flow: | 1.0 ml min⁻¹ |
| UV: | 254 nm |
| Retention times: | |
| Isomer C | 20.3 min |
and
(c) dextrorotatory optical activity,

12. 3,11b-*cis*-Dihydrotetrabenazine according to claim 1 in the form of Isomer D which has:
(a) spectroscopic characteristics as follows:
| ¹H-NMR spectrum (CDCl₃) | ¹³C-NMR spectrum (CDCl₃) | 1R Spectrum (KBr solid) | Mass Spectrum (ES⁺) |
|---|---|---|---|
| 6.68 δ 1H (s); | 147.8 δ; | 3370 cm⁻¹; | MH⁺ 320 |
| 6.58 δ 1H (s); | 147.7 δ; | 2950 cm⁻¹; | |
| 3.92 δ 1H (m); | 130.4 δ; | 2929 cm⁻¹; | |
| 3.84 δ 6H (s); | 127.2 δ; | 1611 cm⁻¹; | |
| 3.15 δ 1H (m); | 112.0 δ; | 1512 cm⁻¹; | |
| 2.87 δ 3H (m); | 108.3 δ; | 1463 cm⁻¹ | |
| 2.43 δ 4H (m); | 72.4 δ; | 1362 cm⁻¹; | |
| 1.81 δ 1H (m); | 61.2 δ; | 1334 cm⁻¹; | |
| 1.64 δ 4H (m); | 58.3 δ; | 1259 cm⁻¹; | |
| 1.21 δ 1H (m); | 56.5 δ; | 1227 cm⁻¹; | |
| 0.94 δ 3H (d); | 56.3 δ; | 1148 cm⁻¹; | |
| 0.89 δ 3H (d) | 52.7 δ; | 1063 cm⁻¹; | |
| | 38.6 δ; | 1024 cm⁻¹; | |
| | 36.7 δ; | 855 cm⁻¹; | |
| | 34.4 δ; | 766 cm⁻¹ | |
| | 29.6 δ; | | |
| | 26.5 δ; | | |
| | 24.4 δ; | | |
| | 22.5 δ | | |
(b) chromatographic characteristics as follows:
| Column: | |
|---|---|
| Chirex (S)-VAL, (R)-NEA, 250 x 4.6mm | |
| Mobile phase: | Hexane : methanol (92:8) |
| Flow: | 1.0 ml min⁻¹ |
| UV: | 254 nm |
| Retention time: | |
| Isomer D | 19.4 min |
and
(c) laevorotatory optical activity.

13. A 3,11b-*cis*-dihydrotetrabenazine as defined in any one of the preceding claims in the form of a free base.

14. A 3,11b-*cis*-dihydrotetrabenazine as defined in any one of claims 1 to 12 in the form of an acid addition salt.

15. A 3,11b-*cis*-dihydrotetrabenazine according to claim 14 wherein the salt is a methane sulphonate salt.

16. A 3,11b-*cis*-dihydrotetrabenazine as defined in any one of the preceding claims for use in medicine or therapy.

17. A 3,11b-*cis*-dihydrotetrabenazine as defined in any one of the preceding claims for use in the treatment of hyperkinetic movement disorders or the treatment of depression.

18. A 3,11b-*cis*-dihydrotetrabenazine for use as defined in claim 17 wherein the hyperkinetic movement disorders is Huntington's disease, hemiballismus, senile chorea, tic, tardive dyskinesia or Tourette's syndrome.

19. A pharmaceutical composition comprising a 3,11b-*cis*-dihydrotetrabenazine as defined in any one of claims 1 to 15 and a pharmaceutically acceptable carrier.

20. The use of a 3,11b-*cis*-dihydrotetrabenazine as defined in any one of claims 1 to 15 for the manufacture of a medicament for the treatment of hyperkinetic movement disorders or the treatment of depression.

21. The use according to claim 20 wherein the medicament is for the treatment of Huntington's disease, hemiballismus, senile chorea, tic, tardive dyskinesia or Tourette's syndrome.

22. A process for preparing a 3,11b-*cis*-dihydrotetrabenazine as defined in any one of claims 5, 6, 9 and 10, which process comprises the reaction of a compound of the formula (II): 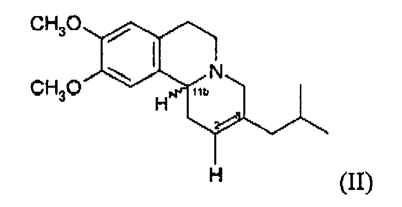 with a reagent or reagents suitable for hydrating the 2,3-double bond in the compound of formula (II) and thereafter where required separating and isolating a desired 3,11b-*cis*-dihydrotetrabenazine isomer form.

23. A process for preparing a 3,11b-*cis*-dihydrotetrabenazine as defined in any one of claims 7, 8, 11 and 12, which process comprises subjecting a compound of the Formula (III): 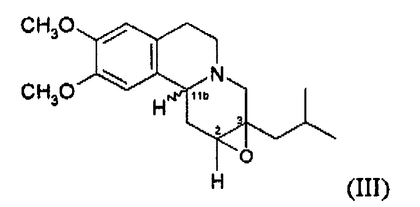 to conditions for ring-opening the 2,3-epoxide group in the compound of the formula (III), and thereafter where required separating and isolating a desired 3,11b-*cis*-dihydrotetrabenazine isomer form.

24. A process for preparing a compound of the formula (III) as defined in claim 23 which process comprises reacting an alkene compound of the formula (II) as defined in claim 22 with an oxidising agent suitable for forming an epoxide group.

25. A process according to claim 24 wherein the oxidising agent is a peroxy acid.

26. A process for preparing a compound of the formula (II) as defined in claim 22 which process comprises dehydrating a 3,11-*trans*-dihydrotetrabenazine with a dehydrating agent such as a phosphorus halide or phosphorus oxyhalide.

27. A compound of the formula (II):

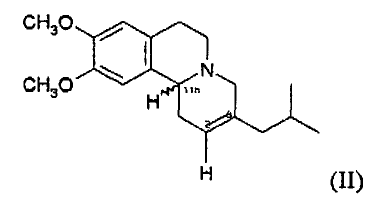

28. A compound of the formula (III):

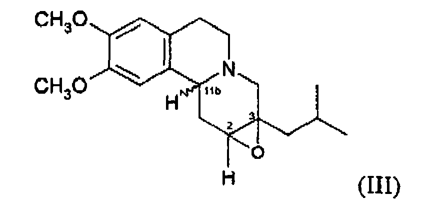

29. A Mosher's acid ester of a compound as defined in any one of claims 1 to 15.

## Patentansprüche

1. 3,11b-*cis*-Dihydrotetrabenazin.

2. 3,11b-*cis*-Dihydrotetrabenazin nach Anspruch 1, das in einer (+)-isomeren Form vorliegt.

3. Zusammensetzung umfassend 3,11b-*cis*-Dihydrotetrabenazin nach Anspruch 1, wobei die Zusammensetzung im Wesentlichen von 3,11b-*trans*-Dihydrotetrabenazin frei ist.

4. Zusammensetzung nach Anspruch 3, wobei das 3,11b-*cis*-Dihydrotetrabenazin ein (+)-Isomer ist.

5. 3,11b-*cis*- Dihydrotetrabenazin nach Anspruch 1, das das 2*S*,3*S*,11b*R*-Isomer ist, das die Formel (Ia): 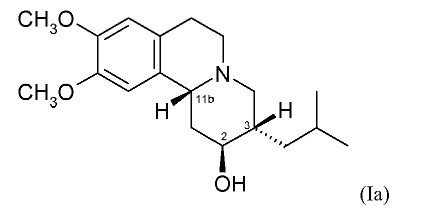 aufweist.

6. 3,11b-*cis*-Dihydrotetrabenazin nach Anspruch 1, das das 2*R*,3*R*,11b*S*-Isomer ist, das die Formel (Ib): 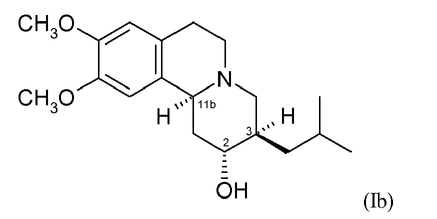 aufweist.

7. 3,11b-*cis*-Dihydrotetrabenazin nach Anspruch 1, das das 2*R*,3*S*,11b*R*-Isomer ist, das die Formel (Ic): 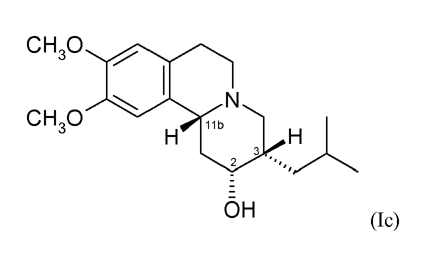 aufweist.

8. 3,11b-*cis*-Dihydrotetrabenazin nach Anspruch 1, das das 2*S*,3*R*,11b*S*-Isomer ist, das die Formel (Id): 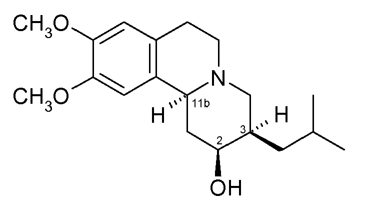 aufweist.

9. 3,11b-*cis*-Dihydrotetrabenazin nach Anspruch 1 in Form von Isomer A, das Folgendes aufweist:
(a) spektroskopische Charakteristiken wie folgt:
| ¹H-NMR-Spektrum | ¹³C-NMR-Spektrum | IR-Spektrum | Massenspektrum |
|---|---|---|---|
| (CDCl₃) | (CDCl₃) | (festes KBr) | (ES⁺) |
| 6,67 δ 1H (s); | 147,7 δ; | 2950 cm⁻¹ | MH⁺ 320 |
| 6,57 δ 1H (s); | 147,6 δ; | 2928 cm⁻¹ | |
| 3,84 δ 6H (s); | 130,5 δ; | 2868 cm⁻¹ | |
| 3,55 δ 1H (br. d); | 127,6 δ; | 2834 cm⁻¹ | |
| 3,08 δ 1H (m); | 112,1 δ; | 1610 cm⁻¹ | |
| 2,79 δ 2H (m); | 108,4 δ; | 1511 cm⁻¹ | |
| 2,55 δ 3H (m); | 70,5 δ; | 1464 cm⁻¹ | |
| 2,17 δ 1H (m); | 57,5 δ; | 1364 cm⁻¹ | |
| 1,72 δ 6H (m); | 56,5 δ; | 1324 cm⁻¹ | |
| 1,02 δ 1H (m); | 56,3 δ**;** | 1258 cm⁻¹ | |
| 0,88 δ 6H (t) | 54,8 δ; | 1223 cm⁻¹ | |
| | 53,2 δ; | 1208 cm⁻¹ | |
| | 40,4 δ; | 1144 cm⁻¹ | |
| | 40,1 δ; | 1045 cm⁻¹ | |
| | 36,0 δ; | 1006 cm⁻¹ | |
| | 28,8 δ; | 870 cm⁻¹ | |
| | 26,2 δ; | 785 cm⁻¹ | |
| | 23,7 δ; | 764 cm⁻¹ | |
| | 22,9 δ | | |
(b) chromatographische Charakteristiken wie folgt:
| Chirale HPLC-Methoden und Aufenthaltszeiten | |
|---|---|
| Säule: | |
| Chirex (S)-VAL, (R)-NEA, 250 x 4,6 mm | |
| Mobile Phase: | Hexan : 1,2-Dichlorethan : Ethanol |
| (36:62:2) | |
| Strömungsgeschwindigkeit: | 1,0 ml min⁻¹ |
| UV: | 254 nm |
| Retentionszeit: | |
| Isomer A | 16,6 min |
und
(c) optische Lävorotationsaktivität.

10. 3,11b-*cis*-Dihydrotetrabenazin nach Anspruch 1 in Form von Isomer B, das Folgendes aufweist:
(a) spektroskopische Charakteristiken wie folgt:
| ¹H-NMR-Spektrum (CDCl₃) | ¹³C-NMR-Spektrum (CDCl₃) | IR-Spektrum (festes KBr) | Massenspektrum (ES⁺) |
|---|---|---|---|
| 6,67 δ 1H (s); | 147,7 δ; | 2950 cm⁻¹ | MH⁺ 320 |
| 6,57 δ 1H (s); | 147,6 δ; | 2928 cm⁻¹ | |
| 3,84 δ 6H (s); | 130,5 δ; | 2868 cm⁻¹ | |
| 3,55 δ 1H (br. d); | 127,6 δ; | 2834 cm⁻¹ | |
| 3,08 δ 1H (m); | 112,1 δ; | 1610 cm⁻¹ | |
| 2,79 δ 2H (m); | 108,4 δ; | 1511 cm⁻¹ | |
| 2,55 δ 3H (m); | 70,5 δ; | 1464 cm⁻¹ | |
| 2,17 δ 1H (m); | 57,5 δ; | 1364 cm⁻¹ | |
| 1,72 δ 6H (m); | 56,5 δ; | 1324 cm⁻¹ | |
| 1,02 δ 1H (m); | 56,3 δ; | 1258 cm⁻¹ | |
| 0,88 δ 6H (t) | 54,8 δ; | 1223 cm⁻¹ | |
| | 53,2 δ; | 1208 cm⁻¹ | |
| | 40,4 δ; | 1144 cm⁻¹ | |
| | 40,1 δ; | 1045 cm⁻¹ | |
| | 36,0 δ; | 1006 cm⁻¹ | |
| | 28,8 δ; | 870 cm⁻¹ | |
| | 26,2 δ; | 785 cm⁻¹ | |
| | 23,7 δ; | 764 cm⁻¹ | |
| | 22,9 δ | | |
(b) chromatographische Characteristiken wie folgt:
| Chirale HPLC-Methoden und Retentionszeit | |
|---|---|
| Säule: | |
| Chirex (S)-VAL, (R)-NEA, 250 x 4,6 mm | |
| Mobile Phase: | Hexan : 1,2-Dichlorethan : ethanol (36:62:2) |
| Strömungsgeschwindigkeit: | 1,0 ml min⁻¹ |
| UV: | 254 nm |
| Retentionszeit: | |
|---|---|
| Isomer B | 15,3 min |
und
(c) optische Dextrorotationsaktivität.

11. 3,11b-*cis*-Dihydrotetrabenazin nach Anspruch I in Form von Isomer C, das Folgendes aufweist:
(a) spektroskopische Charakteristiken wie folgt:
| ¹H-NMR-Spektrum (CDCl₃) | ¹³C-NMR-Spektrum (CDCl₃) | IR-Spektrum (festes KBr) | Massenspektrum (ES⁺) |
|---|---|---|---|
| 6,68 δ 1H (s); | 147,8 δ; | 3370 cm⁻¹ | MH⁺ 320 |
| 6,58 δ 1H (s); | 147,7 δ; | 2950 cm⁻¹ | |
| 3,92 δ 1H (m); | 130,4 δ; | 2929 cm⁻¹ | |
| 3,84 δ 6H (s); | 127,2 δ; | 1611 cm⁻¹ | |
| 3,15 δ 1H (m); | 112,0 δ; | 1512 cm⁻¹ | |
| 2,87 δ 3H (m); | 108,3 δ; | 1463 cm⁻¹ | |
| 2,43 δ 4H (m); | 72,4 δ; | 1362 cm⁻¹ | |
| 1,81 δ 1H (m); | 61,2 δ; | 1334 cm⁻¹ | |
| 1,64 δ 4H (m); | 58,3 δ; | 1259 cm⁻¹ | |
| 1,21 δ 1H (m); | 56,5 δ; | 1227 cm⁻¹ | |
| 0,94 δ 3H (d); | 56,3 δ; | 1148 cm⁻¹ | |
| 0,89 δ 3H (d) | 52,7 δ; | 1063 cm⁻¹ | |
| | 38,6 δ; | 1024 cm⁻¹ | |
| | 36,7 δ; | 855 cm⁻¹ | |
| | 34,4 δ; | 766 cm⁻¹ | |
| | 29,6 δ; | | |
| | 26,5 δ; | | |
| | 24,4 δ; | | |
| | 22,5 δ | | |
(b) chromatographische Charakteristiken wie folgt:
| Säule: | |
|---|---|
| Chirex (S)-VAL, (R)-NEA, 250 x 4,6 mm | |
| Mobile Phase: | Hexan : Ethanol (92:8) |
| Strömungsgeschwindigkeit: | 1,0 ml min⁻¹ |
| UV: | 254 nm |
| Retentionszeit: | |
| Isomer C | 20,3 min |
und
(c) optische Dextrorotationsaktivität.

12. 3,11b-*cis*-Dihydrotetrabenazin nach Anspruch 1 in Form von Isomer D, das Folgendes aufweist:
(a) spektroskopische Charakteristiken wie folgt:
| ¹H-NMR--Spektrum (CDCl₃) | ¹³C-NMR-Spektrum (CDCl₃) | IR-Spektrum (festes KBr) | Massenspektrum (ES⁺) |
|---|---|---|---|
| 6,68 δ 1H (s); | 147,8 δ; | 3370 cm⁻¹ | MH⁺ 320 |
| 6,58 δ 1H (s); | 147,7 δ; | 2950 cm⁻¹ | |
| 3,92 δ 1H (m); | 130,4 δ; | 2929 cm⁻¹ | |
| 3,84 δ 6H (s); | 127,2 δ; | 1611 cm⁻¹ | |
| 3,15 δ 1H (m); | 112,0 δ; | 1512 cm⁻¹ | |
| 2,87 δ 3H (m); | 108,3 δ; | 1463 cm⁻¹ | |
| 2,43 δ 4H (m); | 72,4 δ; | 1362 cm⁻¹ | |
| 1,81 δ 1H (m); | 61,2 δ; | 1334 cm⁻¹ | |
| 1,64 δ 4H (m); | 58,3 δ; | 1259 cm⁻¹ | |
| 1,21 δ 1H (m); | 56,5 δ; | 1227 cm⁻¹ | |
| 0,94 δ 3H (d); | 56,3 δ; | 1148 cm⁻¹ | |
| 0,89 δ 3H (d) | 52,7 δ; | 1063 cm⁻¹ | |
| | 38,6 δ; | 1024 cm⁻¹ | |
| | 36,7 δ; | 855 cm⁻¹ | |
| | 34,4 δ; | 766 cm⁻¹ | |
| | 29,6 δ; | | |
| | 26,5 δ; | | |
| | 24,4 δ; | | |
| | 22,5 δ | | |
(b) chromatographische Charakteristiken wie folgt:
| Säule: | |
|---|---|
| Chirex (S)-VAL, (R)-NEA, 250 x 4,6 mm | |
| Mobile Phase: Hexan : | Ethanol (92:8) |
| Strömungsgeschwindigkeit: | 1,0 ml min⁻¹ |
| UV: | 254 nm |
| Retentionszeit: | |
| Isomer D | 19,4 min |
und
(c) optische Lävorotationsaktivität.

13. 3,11b-*cis*-Dihydrotetrabenazin, wie in einem der vorhergehenden Ansprüche definiert, in Form einer freien Base.

14. 3,11b-*cis*-Dihydrotetrabenazin, wie in einem der Ansprüche 1 bis 12 definiert, in Form eines sauren Additionssalzes.

15. 3,11b-*cis*-Dihydrotetrabenazin, nach Anspruch 14, wobei das Salz ein Methansulfonatsalz ist.

16. 3,llb-cis-Dihydrotetrabenazin, wie in einem der vorhergehenden Ansprüche definiert, zur Verwendung in der Medizin oder bei der Therapie.

17. 3,11b-*cis*-Dihydrotetrabenazin, wie in einem der vorhergehenden Ansprüche definiert, zur Verwendung bei der Behandlung von hyperkinetischen Bewegungsstörungen oder der Behandlung von Depression.

18. 3,11b-*cis*-Dihydrotetrabenazin zur Verwendung wie in Anspruch 17 definiert, wobei die hyperkinetischen Bewegungsstörungen Chorea Huntington, Hemiballismus, senile Chorea, Muskelzucken, Dyskinesia tardive oder Tourette-Syndrom sind.

19. Pharmazeutische Zusammensetzung umfassend ein 3,11b-*cis*-Dihydrotetrabenazin, wie in einem der Ansprüche 1 bis 15 definiert, und einen pharmazeutisch akzeptablen Träger.

20. Verwendung eines 3,11b-*cis*-Dihydrotetrabenazins, wie in einem der Ansprüche 1 bis 15 definiert, für die Herstellung eines Medikaments für die Behandlung von hyperkinetischen Bewegungsstörungen oder die Behandlung von Depression.

21. Verwendung nach Anspruch 20, wobei das Medikament der Behandlung von Chorea Huntington, Hemiballismus, seniler Chorea, Muskelzucken, Dyskinesia tardive oder Tourette-Syndrom dient.

22. Verfahren für die Herstellung von 3,11b-*cis*-Dihydrotetrabenazin, wie in einem der Ansprüche 5, 6, 9 und 10 definiert, welches Verfahren die Reaktion einer Verbindung der Formel (II): 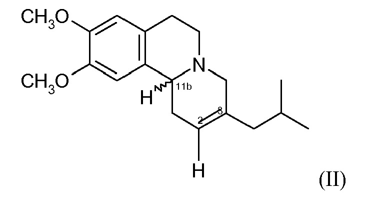 mit einem Reagens oder Reagentien, die für das Hydratisieren der 2,3-Doppelbindung in der Verbindung der Formel (II) geeignet ist bzw. sind, und daraufhin, falls erforderlich, das Trennen und Isolieren einer erwünschten 3,11b-cis-Dihydrotetrabenazin-Isomerform umfasst.

23. Verfahren für die Herstellung eines 3,11b-*cis*-Dihydrotetrabenazins, wie in einem der Ansprüche 7, 8, 11 and 12 definiert, welches Verfahren das Unterwerfen einer Verbindung der Formel (III): 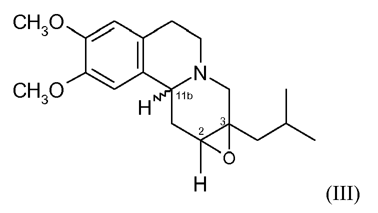 Bedingungen gegenüber für die Ringöffung der 2,3-Epoxidgruppe in der Verbindung der Formel (III) und daraufhin, falls erforderlich, das Trennen und Isolieren einer erwünschten 3,11b-*cis*-Dihydrotetrabenazin-Isomerform umfasst.

24. Verfahren für die Herstellung einer Verbindung der Formel (III), wie in Anspruch 23 definiert, welches Verfahren das Reagieren einer Alkenverbindung der Formel (II), wie in Anspruch 22 definiert, mit einem Oxidationsmittel, das zum Bilden einer Epoxidgruppe geeignet ist, umfasst.

25. Verfahren nach Anspruch 24, wobei das Oxidationsmittel eine Peroxysäure ist.

26. Verfahren für die Herstellung einer Verbindung der Formel (II), wie in Anspruch 22 definiert, welches Verfahren das Dehydratisieren eines 3,11-*trans*-Dihydrotetrabenazins mit einem Dehydratisiermittel, wie beispielsweise einem Phosphorhalogenid oder Phosphoroxyhalogenid, umfasst.

27. Verbindung der Formel (II):

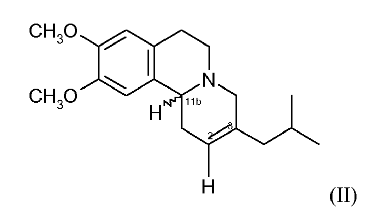

28. Verbindung der Formel (III):

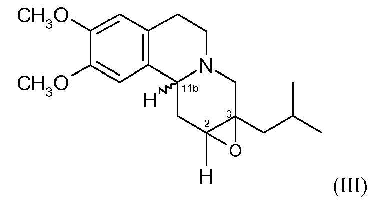

29. Mosher-Säureester einer Verbindung, wie in einem der Ansprüche 1 bis 15 definiert.

## Revendications

1. 3,11b-*cis*-Dihydrotétrabénazine.

2. 3,11b-*cis*-Dihydrotétrabénazine conforme à la revendication 1 qui est sous une forme isomère (+).

3. Une composition comportant de la 3,11b-*cis*-dihydrotétrabénazine conformément à la revendication 1, ladite composition étant sensiblement exempte de 3,11b-*trans-*dihydrotétrabénazine

4. Une composition conforme à la revendication 3 dans laquelle la 3,11b-*cis-*dihydrotétrabénazine est un isomère (+).

5. 3,11b-*cis*-Dihydrotétrabénazine conforme à la revendication 1 qui est l'isomère 2*S*,3*S*,11b*R* ayant la formule (Ia) :

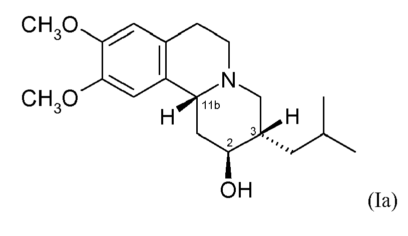

6. 3,11b-*cis*-Dihydrotétrabénazine conforme à la revendication 1 qui est l'isomère 2*R*,3*R*,11b*S* ayant la formule (Ib) :

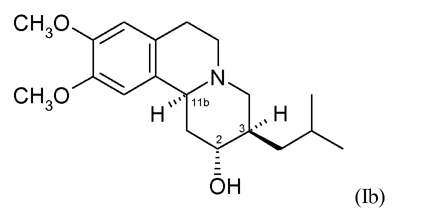

7. 3,11b-*cis*-Dihydrotétrabénazine conforme à la revendication 1 qui est l'isomère 2*R*,3*S*,11b*R* ayant la formule (Ic) :

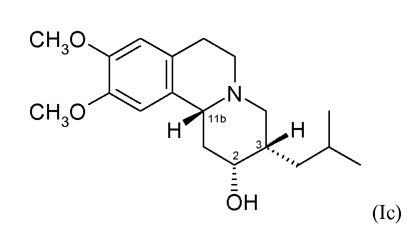

8. 3,11b-*cis*-Dihydrotétrabénazine conforme à la revendication 1 qui est l'isomère 2*S*,3*R*,11b*S* ayant la formule (Id) :

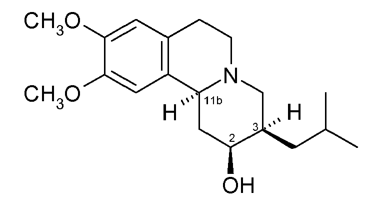

9. 3,11b-*cis*-Dihydrotétrabénazine conforme à la revendication 1 sous la forme d'Isomère A qui possède :
(a) les caractéristiques spectroscopiques suivantes :
| Spectre ¹H-NMR (CDCl₃) | Spectre ¹³C-NMR (CDCl₃) | Spectre IR (KBr solide) | Spectre de masse (ES⁺) |
|---|---|---|---|
| 6,67 δ 1H (s) ; | 147,7 δ ; | 2 950 cm⁻¹ | MH⁺ 320 |
| 6,57 δ 1H (s) ; | 147,6 δ ; | 2 928 cm⁻¹ | |
| 3,84 δ 6H (s) ; | 130,5 δ ; | 2 868 cm⁻¹ | |
| 3,55 δ 1H (br. d) ; | 127,6 δ ; | 2 834 cm⁻¹ | |
| 3,08 δ 1H (m) ; | 112,1 δ ; | 1 610 cm⁻¹ | |
| 2,79 δ 2H (m) ; | 108,4 δ ; | 1 511 cm⁻¹ | |
| 2,55 δ 3H (m) ; | 70,5 δ ; | 1 464 cm⁻¹ | |
| 2,17 δ 1H (m) ; | 57,5 δ ; | 1 364 cm⁻¹ | |
| 1,72 δ 6H (m) ; | 56,5 δ ; | 1 324 cm⁻¹ | |
| 1,02 δ 1H (m) ; | 56,3 δ ; | 1 258 cm⁻¹ | |
| 0,88 δ 6H (t) | 54,8 δ ; | 1 223 cm⁻¹ | |
| | 53,2 δ ; | 1 208 cm⁻¹ | |
| | 40,4 δ ; | 1 144 cm⁻¹ | |
| | 40,1 δ ; | 1 045 cm⁻¹ | |
| | 36,0 δ ; | 1 006 cm⁻¹ | |
| | 28,8 δ ; | 870 cm⁻¹ | |
| | 26,2 δ ; | 785 cm⁻¹ | |
| | 23,7 δ ; | 764 cm⁻¹ | |
| | 22,9 δ | | |
(b) les caractéristiques chromatographiques suivantes :
| Méthodes CLHP Chirales et temps de rétention | |
|---|---|
| Colonne : | |
| Chirex (S)-VAL, (R)-NEA, 250 x 4,6 mm | |
| Phase mobile : | Hexane :1,2-dichloroéthane : éthanol |
| (36:62:2) | |
| Débit : | 1,0 ml min⁻¹ |
| UV : | 254 nm |
| Temps de rétention : | |
| Isomère A | 16,6 min |
et
(c) une activité optique lévogyre.

10. 3,11b-*cis*-Dihydrotétrabénazine conforme à la revendication 1 sous la forme d'Isomère B qui possède :
(a) les caractéristiques spectroscopiques suivantes :
| Spectre ¹H-NMR (CDCl₃) | Spectre ¹³C-NMR (CDCl₃) | Spectre IR (KBr solide) | Spectre de masse (ES⁺) |
|---|---|---|---|
| 6,67 δ 1H (s) ; | 147,7 δ ; | 2 950 cm⁻¹ | MH⁺ 320 |
| 6,57 δ 1H (s) ; | 147,6 δ ; | 2 928 cm⁻¹ | |
| 3,84 δ 6H (s) ; | 130,5 δ ; | 2 868 cm⁻¹ | |
| 3,55 δ 1H (br. d) ; | 127,6 δ ; | 2 834 cm⁻¹ | |
| 3,08 δ 1H (m) ; | 112,1 δ ; | 1 610 cm⁻¹ | |
| 2,79 δ 2H (m) ; | 108,4 δ ; | 1 511 cm⁻¹ | |
| 2,55 δ 3H (m) ; | 70,5 δ ; | 1 464 cm⁻¹ | |
| 2,17 δ 1H (m) ; | 57,5 δ ; | 1 364 cm⁻¹ | |
| 1,72 δ 6H (m) ; | 56,5 δ ; | 1 324 cm⁻¹ | |
| 1,02 δ 1H (m) ; | 56,3 δ ; | 1 258 cm⁻¹ | |
| 0,88 δ 6H (t) | 54,8 δ ; | 1 223 cm⁻¹ | |
| | 53,2 δ ; | 1 208 cm⁻¹ | |
| | 40,4 δ ; | 1 144 cm⁻¹ | |
| | 40,1 δ ; | 1 045 cm⁻¹ | |
| | 36,0 δ ; | 1006 cm⁻¹ | |
| | 28,8 δ ; | 870 cm⁻¹ | |
| | 26,2 δ ; | 785 cm⁻¹ | |
| | 23,7 δ ; | 764 cm⁻¹ | |
| | 22,9 δ | | |
(b) les caractéristiques chromatographiques suivantes :
| Colonne : | |
|---|---|
| Chirex (S)-VAL, (R)-NEA, 250 x 4,6 mm | |
| Phase mobile : | Hexane : 1,2-dichloroéthane : éthanol |
| (36:62:2) | |
| Débit : | 1,0 ml min⁻¹ |
| UV: | 254 nm |
| Temps de rétention : | |
| Isomère B | 15,3 min |
et
(c) une activité optique dextrogyre

11. 3,11b-*cis*-Dihydrotétrabénazine conforme à la revendication 1 sous la forme d'Isomère C qui possède :
(a) les caractéristiques spectroscopiques suivantes :
| Spectre ¹H-NMR (CDCl₃) | Spectre ¹³C-NMR (CDCl₃) | Spectre IR (KBr solide) | Spectre de masse (ES⁺) |
|---|---|---|---|
| 6,68 δ 1H (s) ; | 147,8 δ ; | 3 370 cm⁻¹ | MH⁺ 320 |
| 6,58 δ 1H (s) ; | 147,7 δ ; | 2 950 cm⁻¹ | |
| 3,92 δ 1H (m) ; | 130,4 δ ; | 2 929 cm⁻¹ | |
| 3,84 δ 6H (s) ; | 127,2 δ ; | 1 611 cm⁻¹ | |
| 3,15 δ 1H (m) ; | 112,0 δ ; | 1 512 cm⁻¹ | |
| 2,87 δ 3H (m) ; | 108,3 δ ; | 1 463 cm⁻¹ | |
| 2,43 δ 4H (m) ; | 72,4 δ ; | 1 362 cm⁻¹ | |
| 1,81 δ 1H (m) ; | 61,2 δ ; | 1 334 cm⁻¹ | |
| 1,64 δ 4H (m) ; | 58,3 δ ; | 1 259 cm⁻¹ | |
| 1,21 δ 1H (m) ; | 56,5 δ ; | 1 227 cm⁻¹ | |
| 0,94 δ 3H (d) ; | 56,3 δ ; | 1 148 cm⁻¹ | |
| 0,89 δ 3H (d) | 52,7 δ ; | 1 063 cm⁻¹ | |
| | 38,6 δ ; | 1 024 cm⁻¹ | |
| | 36,7 δ ; | 855 cm⁻¹ | |
| | 34,4 δ ; | 766 cm⁻¹ | |
| | 29,6 δ ; | | |
| | 26,5 δ ; | | |
| | 24,4 δ ; | | |
| | 22,5 δ | | |
(b) les caractéristiques chromatographiques suivantes :
| Colonne : | |
|---|---|
| Chirex (S)-VAL, (R)-NEA, 250 x 4,6 mm | |
| Phase mobile : | Hexane : éthanol (92:8) |
| Débit : | 1,0 ml min⁻¹ |
| UV: | 254 nm |
| Temps de rétention : | |
| Isomère C | 20,3 min |
et
(c) une activité optique dextrogyre

12. 3,11b-*cis*-Dihydrotétrabénazine conforme à la revendication 1 sous la forme d'Isomère D qui possède :
(a) les caractéristiques spectroscopiques suivantes :
| Spectre ¹H-NMR (CDCl₃) | Spectre ¹³C-NMR (CDCl₃) | Spectre IR (KBr solide) | Spectre de masse (ES⁺) |
|---|---|---|---|
| 6,68 δ 1H (s) ; | 147,8 δ ; | 3 370 cm⁻¹ | MH⁺ 320 |
| 6,58 δ 1H (s) ; | 147,7 δ ; | 2 950 cm⁻¹ | |
| 3,92 δ 1H (m) ; | 130,4 δ ; | 2 929 cm⁻¹ | |
| 3,84 δ 6H (s) ; | 127,2 δ ; | 1 611 cm⁻¹ | |
| 3,15 δ 1H (m) ; | 112,0 δ ; | 1 512 cm⁻¹ | |
| 2,87 δ 3H (m) ; | 108,3 δ ; | 1 463 cm⁻¹ | |
| 2,43 δ 4H (m) ; | 72,4 δ ; | 1 362 cm⁻¹ | |
| 1,81 δ 1H (m) ; | 61,2 δ ; | 1 334 cm⁻¹ | |
| 1,64 δ 4H (m) ; | 58,3 δ ; | 1 259 cm⁻¹ | |
| 1,21 δ 1H (m) ; | 56,5 δ ; | 1 227 cm⁻¹ | |
| 0,94 δ 3H (d) ; | 56,3 δ ; | 1 148 cm⁻¹ | |
| 0,89 δ 3H (d) | 52,7 δ ; | 1 063 cm⁻¹ | |
| | 38,6 δ ; | 1 024 cm⁻¹ | |
| | 36,7 δ ; | 855 cm⁻¹ | |
| | 34,4 δ ; | 766 cm⁻¹ | |
| | 29,6 δ ; | | |
| | 26,5 δ ; | | |
| | 24,4 δ ; | | |
| | 22,5 δ | | |
(b) les caractéristiques chromatographiques suivantes :
| Méthodes CLHP Chirales et temps de rétention | |
|---|---|
| Colonne : | |
| Chirex (S)-VAL, (R)-NEA, 250 x 4,6 mm | |
| Phase mobile : | Hexane : éthanol (92:8) |
| Débit : | 1,0 ml min⁻¹ |
| UV : | 254 nm |
| Temps de rétention : | |
| Isomère D | 19,4 min |
et
(c) une activité optique lévogyre.

13. Une 3,11b-*cis*-dihydrotétrabénazine telle que définie dans l'une quelconque des précédentes revendications sous la forme d'une base libre.

14. Une 3,11b-*cis*-dihydrotétrabénazine telle que définie dans l'une quelconque des revendications 1 à 12 sous la forme d'un sel d'addition acide.

15. Une 3,11b-*cis*-dihydrotétrabénazine conforme à la revendication 14 dans laquelle le sel est un sel de méthanesulfonate.

16. Une 3,11b-*cis*-dihydrotétrabénazine telle que définie dans l'une quelconque des précédentes revendications pour un usage médical ou thérapeutique.

17. Une 3,11b-*cis*-dihydrotétrabénazine telle que définie dans l'une quelconque des revendications précédentes pour un usage dans le traitement des troubles hyperkinétiques ou de la dépression.

18. Une 3,116-*cis*-dihydrotétrabénazine pour un usage tel que défini à la revendication 17 dans laquelle les troubles hyperkinétiques sont la maladie de Huntington, l'hémiballisme, la chorée sénile, les tics, la dyskinésie tardive ou le syndrome de Tourette.

19. Composition pharmaceutique comprenant une 3,11b-*cis*-dihydrotétrabénazine telle que définie dans l'une quelconque des revendications 1 à 15 et un vecteur de qualité pharmaceutique.

20. L'utilisation d'une 3,11b-*cis*-dihydrotétrabénazine telle que définie dans l'une quelconque des revendications 1 à 15 pour la production d'un médicament pour le traitement des troubles hyperkinétiques ou de la dépression.

21. L'utilisation conforme à la revendication 20 dans laquelle le médicament est pour le traitement de la maladie de Huntington, de l'hémiballisme, de la chorée sénile, des tics, de la dyskinésie tardive ou du syndrome de Tourette.

22. Un processus pour préparer une 3,11b-*cis*-dihydrotétrabénazine tel que défini dans l'une quelconque des revendications 5, 6, 9 et 10 ledit processus comprenant la réaction d'un composé de la formule (II) : 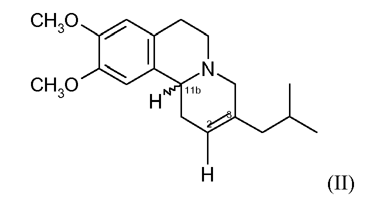 avec un ou plusieurs réactif(s) convenant à l'hydratation de la double liaison 2,3 dans le composé de la formule (II) et, par la suite s'il y a lieu, à la séparation et à l'isolation d'une forme d'isomère de 3,11b-*cis*-dihydrotétrabénazine.

23. Un processus pour préparer une 3,11b-*cis*-dihydrotétrabénazine tel que défini dans l'une quelconque des revendications 7, 8, 11 et 12 ledit processus comprenant l'exposition d'un composé de la formule (III) : 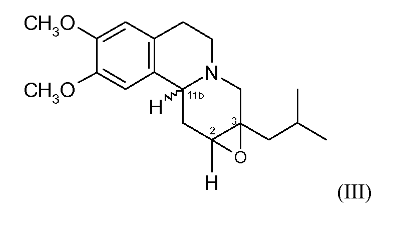 à des conditions pour l'ouverture de noyau du groupe époxyde 2,3 dans le composé de la formule (III) et, par la suite s'il y a lieu, à la séparation et à l'isolation d'une forme d'isomère de 3,11b-*cis*-dihydrotétrabénazine.

24. Un processus de préparation d'un composé de la formule (III) tel que défini à la revendication 23, ledit processus comprenant la réaction d'un composé alkène de la formule (II) tel que défini à la revendication 22 avec un agent oxydant convenant à la formation d'un groupe époxyde.

25. Un processus conforme à la revendication 24 dans lequel l'agent oxydant est un acide péroxy.

26. Processus de préparation d'un composé de la formule (II) tel que défini à la revendication 22 ledit processus comprenant la déshydratation d'une 3,11-*trans-*dihydrotétrabénazine avec un agent déshydratant comme un halogénure de phosphore ou un oxyhalogénure de phosphore.

27. Un composé de la formule (II) :

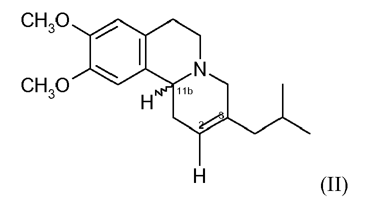

28. Un composé de la formule (III) :

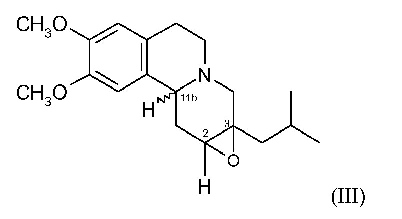

29. Un ester d'acide de Mosher d'un composé tel que défini dans l'une quelconque des revendications 1 à 15.
